Europäisches Patentamt

European Patent Office

Office européen des brevets

(11) Numéro de publication: **0 068 981**
**B1**

(19)

(12) # FASCICULE DE BREVET EUROPEEN

(45) Date de publication du fascicule du brevet:
**31.07.85**

(21) Numéro de dépôt: **82401099.5**

(22) Date de dépôt: **17.06.82**

(51) Int. Cl.⁴: **C 10 L 1/04,** C 07 C 11/08,
C 07 C 7/00, C 10 G 29/00

(54) Procédé d'obtention conjointe de butène-1 de haute pureté et de supercarburant à partir d'une coupe C4 oléfinique.

(30) Priorité: 26.06.81 FR 8112795
02.07.81 FR 8113211
08.12.81 FR 8123065

(43) Date de publication de la demande:
**05.01.83 Bulletin 83/1**

(45) Mention de la délivrance du brevet:
**31.07.85 Bulletin 85/31**

(84) Etats contractants désignés:
**BE DE GB IT NL**

(56) Documents cités:
**FR - A - 2 401 122**
**GB - A - 595 827**
**GB - A - 2 006 263**
**GB - A - 2 068 408**
**US - A - 3 814 679**

(73) Titulaire: **INSTITUT FRANCAIS DU PETROLE, 4, Avenue de Bois-Préau, F-92502 Ruell-Malmaison (FR)**

(72) Inventeur: **Juguin, Bernard, 46, avenue du Stade, F-92500 Ruell-Malmaison (FR)**
Inventeur: **Cosyns, Jean, 50, route d'Herbeville, F-78580 Maule (FR)**
Inventeur: **Miquel, Jean, 5, rue Fragonard, F-75017 Paris (FR)**

EP 0 068 981 B1

BUNDESDRUCKEREI BERLIN

## Description

La présente invention concerne un procédé d'obtention conjointe de butène-1 de haute pureté supérieure à 99%, de supercarburant, et éventuellement d'éthylène et de propène, et de combustible pour carburéacteur, procédé rendu possible par une combinaison judicieuse de plusieurs étapes. La charge initiale est généralement une coupe oléfinique $C_4$ en provenance par exemple d'une zone de craquage à la vapeur.

Le procédé selon l'invention est adapté aux pétrochimistes possédant une unité de craquage à la vapeur d'eau, et permet de mieux valoriser les coupes butènes, le plus souvent excédentaires.

Le procédé selon l'invention permet en particulier d'obtenir:

1) du butène-1 de haute pureté avec des rendements très élevés; la teneur en butène-1 à la sortie du procédé est nettement supérieure à celle du butène-1 initialement contenu dans la charge de départ,

2) du supercarburant,

3) du combustible pour carburéacteur de grandes qualités et éventuellement de l'éthylène et du propène.

Le procédé selon l'invention est caractérisé en ce que:

a) une coupe $C_4$ oléfinique, qui généralement à ce stade renferme de l'isobutane, du n-butane, du butène-1, des butènes-2, de l'isobutène et éventuellement du butadiène (en général moins de 2% et de préférence moins de 0,7% en poids de butadiène) est soumise à une polymérisation-dismutation au cours de laquelle d'une part, l'isobutène de dite coupe est transformé en partie au moins en une essence, et d'autre part l'essence ainsi produite est soumise au moins en partie à une réaction partielle de dismutation de façon à recueillir d'une part une coupe type base pour carburéacteur et d'autre part des butènes-2 et surtout du butène-1 produits par ladite dismutation,

b) l'effluent soutiré de la zone de polymérisation-dismutation est soumis à un fractionnement au cours duquel on recueille d'une part un mélange ($\alpha$) d'essence et de base pour carburéacteur, et d'autre part une fraction ($\beta$) essentiellement constituée d'isobutane, de n-butane, de butène-1, de butènes-2, d'une légère portion d'isobutène (généralement inférieure à 5% et de préférence à 2% en poids),

c) la coupe ($\beta$) obtenue à l'étape (b) est envoyée dans une zone de polymérisation sélective dite polymérisation de finition, dans laquelle plus de 90% de l'isobutène résiduel est transformé en essence,

d) l'effluent soutiré de la zone dite polymérisation de finition effectuée à l'étape (c) est soumis à un fractionnement au cours duquel on recueille d'une part une coupe ($\gamma$) constituée en majeure partie d'essence renfermant notamment un mélange de dimères et de trimères de l'isobutène et des butènes-1 et -2, et d'autre part une fraction ($\delta$) essentiellement constituée d'isobutane, de n-butane, de butène-1, de butènes-2, d'une portion mineure d'isobutène (généralement inférieure à 0,3% et de préférence à 0,15% en poids), et de traces de butadiène (généralement inférieures à 100 parties par million),

e) la coupe ($\delta$) issue l'étape (d) est envoyée dans une zone d'hydrogénation sélective de manière à ce que sa teneur en butadiène soit amenée au maximum à 10 parties par million en poids par rapport au butène-1 de la coupe ($\delta$),

f) l'effluent issu de l'étape d'hydrogénation sélective (e), est envoyé dans une zone de déisobutanisation en vue d'éliminer la majeure partie de l'isobutane qu'il contenait,

g) ladite fraction ainsi déisobutanisée est soumise à un fractionnement au cours duquel on recueille, d'une part une coupe renfermant en majeure partie des butènes-2 et du n-butane, et d'autre part une fraction constituée d'au minimum 99% en poids de butène-1, et en ce que le mélange ($\alpha$) constitué par l'essence et la base pour carburéacteur issue de l'étape (b), est mélangé avec la coupe ($\gamma$) d'essence provenant de l'étape (d).

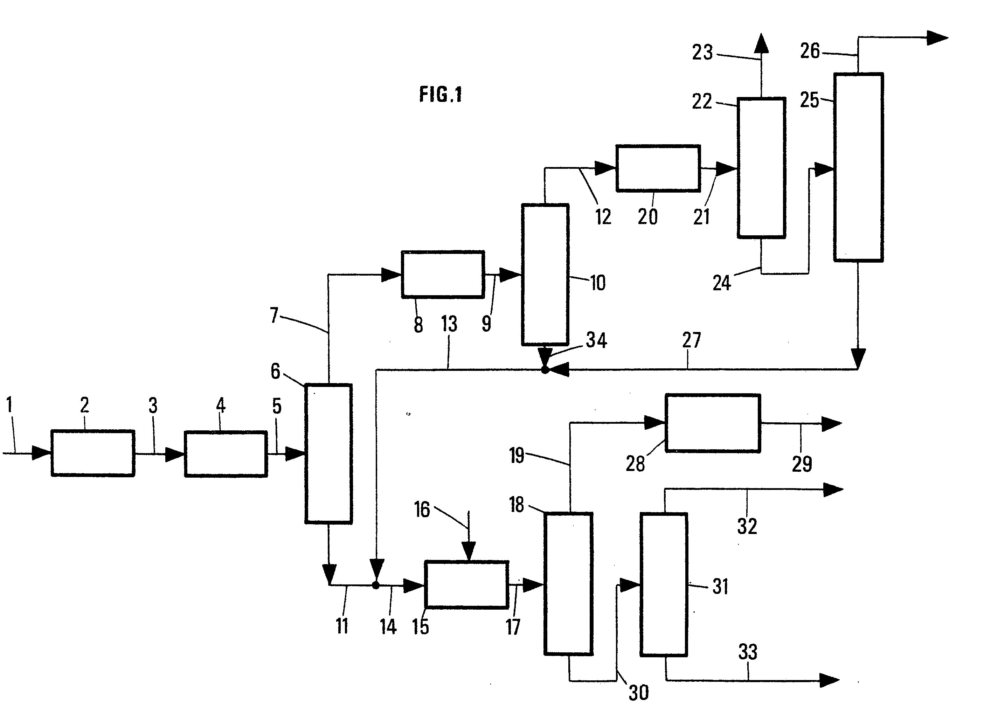

Les trois figures 1 à 3 illustrent 3 modes particuliers de l'invention.

Le procédé selon la figure 1 est caractérisé en ce que:

a) Une coupe $C_4$ oléfinique, qui à ce stade renferme de l'isobutane, du n-butane, du butène-1, des butènes-2, de l'isobutène et moins de 2% de butadiène en poids (et de préférence moins de 0,7% en poids de butadiène), est éventuellement d'abord envoyée dans une zone de séchage (séchage réalisé du façon conventionelle, par exemple par passage de la coupe sur un tamis moléculaire, de préférence un tamis de type 3A), puis est soumise à une polymérisation dismutation au cours de laquelle d'une part, l'isobutène de la dite coupe est transformé en partie au moins en une essence, et d'autre part l'essence ainsi produite est soumise au moins en partie à une réaction partielle de dismutation de façon à recueillir d'une part une coupe type base pour carburéacteur, et d'autre part des butènes-2 et surtout du butène-1 produits par la dite dismutation,

**0 068 981**

b) l'effluent soutiré de la zone de polymérisation-dismutation est soumis à un fractionnement au cours duquel on recueille d'une part un mélange ($\alpha$) d'essence et de base pour carburéacteur, et d'autre part une fraction ($\beta$) essentiellement constituée d'isobutane, de n-butane, de butène-1, de butènes-2, de moins de 5% en poids d'isobutène (et de préférence inférieure à 2% en poids), et de moins de 2000 ppm de butadiène (et de préférence inférieure à 500 parties par million),

c) la coupe ($\beta$) obtenue à l'étape (b) est envoyée dans une zone de polymérisation sélective dite polymérisation de finition, dans laquelle plus de 90% de l'isobutène résiduel est transformé en essence, en ne transformant en essence qu'une très faible portion de butène-1 et de butènes-2,

d) l'effluent soutiré de la zone dite polymérisation de finition effectuée à l'étape (c) est soumis à un fractionnement au cours duquel on recueille d'une part une coupe ($\gamma$) constituée en majeure partie d'essence renfermant notamment un mélange de dimères et de trimères de l'isobutène et des butènes-1 et -2, et d'autre part une fraction ($\delta$) essentiellement constituée d'isobutane, de n-butane, de butène-1, de butènes-2, de moins de 0,3% en poids d'isobutène (et de préférence inférieure à 0,15% en poids), et moins de 100 ppm de butadiène,

e) la coupe ($\delta$) issue de l'étape (d) est envoyée dans une zone d'hydrogénation sélective de manière à ce que sa teneur en butadiène soit amenée au maximum à 10 parties par million en poids par rapport au butène-1 de la coupe ($\delta$),

f) l'effluent issu de l'étape d'hydrogénation sélective (e), est envoyé dans une zone de déisobutanisation en vue d'éliminer la majeure partie l'isobutane qu'il contenait,

g) la dite fraction ainsi déisobutanisée est soumise à un fractionnement au cours duquel on recueille, d'une part une coupe renfermant en majeure partie des butènes-2 et du n-butane, et d'autre part une fraction constituée d'au minimum 99% en poids de butène-1, cette coupe butène-1 convenant parfaitement pour être utilisée, notamment, directement en tant que comonomère dans la production de polyéthylène haute densité,

h) le mélange ($\alpha$) constitué par l'essence et la base pour carburéacteur issue de l'étape (b), est mélangé avec la coupe ($\gamma$) d'essence provenant de l'étape (d) et avec la coupe $C_4$ riche en butènes-2 et en n-butane en provenance de l'étape (g), le nouveau mélange subsequent étant ensuite soumis à une hydrogénation généralement totale, (i) l'effluent d'hydrogénation de l'étape (h) étant soumis à un ou plusieurs fractionnements en vue de recueillir une coupe de n-butane (qui généralement est envoyée dans une zone de craquage à la vapeur d'eau, par exermple celle d'où peut provenir la coupe $C_4$ traitée conformément à l'invention, où elle sera tranformée principalement en éthylène et propène: le n-butane forme en effet avec le n-hexane la charge conduisant aux meilleurs rendements en éthylène, après l'éthane) une coupe essence de haut indice d'octane qui peut être envoyée dans un pool essence, et une coupe combustible pour carburéacteur, dont les qualités sont excellentes.

Au cours de la réaction de polymérisation-dismutation de l'étape (a), l'isobutène est converti essentiellement en une essence constituée essentiellement de dimères et trimères de l'isobutène. La coupe type base pour carburéacteur contient en majeure partie des trimères et tétramères de l'isobutène.

Dans la zone dite de polymérisation de finition, l'isobutène et une petite partie des butènes-1 et -2, sont convertis en dimères et trimères de l'isobutène.

Dans la zone dite de polymérisation-dismutation, les conditions sont telles que l'isobutène réagit, jusqu'à des taux de conversion en essence supérieurs à 95%, en poids, tandis que, non seulement les butènes-1 et -2 ne sont par transformés, mais leur concentration se trouve augmentée par la réaction de dismutation d'une partie de l'essence formée à partir de l'isobutène.

Dans la zone dite de polymérisation de finition, les conditions sont telles que l'isobutène réagit jusqu'à des taux de conversion supérieurs à 90% en poids tandis que les conversions globales des butènes normaux (butène-1 et butènes-2 cis et trans), restent inférieures ou égales à 10% en poids et de préférence inférieures à 7%.

Les réactions de polymérisation-dismutation sont effectuées en phase liquide, en présence d'un catalyseur disposé par exemple sous forme d'un lit fixe, à une température d'environ 10 à 120°C, sous une pression de 0,3 à 10 MPa (de préférence, la température est d'environ 30 à 80°C et la pression est de 0,4 à 3 MPa), avec un débit d'hydrocarbures liquides (Vitesse spatiale) d'environ 0,05 à 2 volumes par volume de catalyseur et par heure.

La réaction dite de polymérisation de finition est également effectuée en phase liquide, en présence d'un catalyseur disposé par exemple sous forme d'un lit fixe, à une température d'environ 30 à 200°C, sous une pression d'environ 0,5 à 20 MPa (de préférence, la température est d'environ 50 à 150°C et la pression est de 1 à 10 MPa), avec un débit d'hydrocarbures liquides (vitesse spatiale) d'environ 0,2 à 4 volumes par volume de catalyseur et par heure.

Les catalyseurs de nature acide sont choisis parmi les silice-alumines, les silice-magnésies, les bore-alumines, l'acide phosphorique sur kieselgurh ou sur silice ou sur quartz et parmi des catalyseurs du type »acide phosphorique solide«, c'est à dire un catalyseur constitué d'une matière siliceuse à grand pouvoir absorbant imprégnée d'une proportion élevée d'acide phosphorique, des mélanges de gel d'alumine et de thorine co-précipités ou non, avec éventuellement des additions d'oxyde de

3

chrôme, d'oxyde de zinc, ou d'un métal équivalent. On peut encore choisir des catalyseurs obtenus par traitement d'alumine de transition au moyen d'un dérivé acide du fluor, avec éventuellement addition d'un ester silicique. De préférence, on utilise une silice alumine dont la teneur en silice est comprise entre 60 et 95% en poids et de préférence entre 70 et 90%, ayant comme additif entre 0,1 et 5% en poids d'oxyde de chrôme et/ou d'oxyde de zinc. La présence d'oxyde de chrôme est préférée pour la polymérisation-dismutation, celle d'oxyde de zinc pour la polymérisation de finition.

La réaction d'hydrogénation sélective du butadiène est effectuée, en présence d'un catalyseur disposé par exemple sous forme d'un lit fixe, le dit catalyseur étant constitué par des métaux ou des associations de métaux hydrogénants déposés sur un support sensiblement neutre, par exemple un catalyseur $Pd/Al_2O_3$ ou $Pd + Ag/Al_2O_3$. La température de réaction sera comprise entre 10 et 100°C, sous une pression de 0,1 à 3 MPa (de préférence, la température est d'environ 20 à 60°C et la pression est de 0,5 à 1,5 MPa), avec un débit d'hydrocarbures liquides (vitesse spatiale) d'environ 2 à 20 volumes par volume de catalyseur et par heure, le rapport molaire hydrogène/butadiène, à l'entrée du réacteur, étant compris entre 0,5 et 5.

La réaction d'hydrogénation du mélange de butènes-2, d'essence et de base type combustible pour carburéacteur est effectuée, en présence d'un catalyseur disposé par exemple sous forme d'un lit fixe, le dit catalyseur étant constitué par un métal hydrogénant déposé sur un support sensiblement neutre, par exemple le catalyseur LD 265 commercialisé par la Société PROCATALYSE. La température de réaction est comprise entre 150 et 400°C, sous une pression de 2 à 10 MPa (de préférence la température est d'environ 220 à 300°C et la pression de 4 à 6 MPa), avec un débit d'hydrocarbures liquides (vitesse spatiale) d'environ 1 à 5 volumes par volume de catalyseur et par heure, le rapport molaire Hydrogène/Hydrocarbures à l'entrée du réacteur étant compris entre 2 et 10.

## La figure 1 illustre ainsi l'invention

La charge oléfinique $C_4$ est introduite par la conduite 1 dans une zone facultative de séchage 2, d'où elle est soutirée par la conduite 3 dans la zone de polymérisation-dismutation 4. L'effluent de la zone de polymérisation-dismutation est acheminé par la conduite 5 dans la zone de fractionnement 6. Par la conduite 11, on recueille essentiellement un mélange de dimères, trimères et tétramères de l'isobutène, c'est-à-dire un mélange de supercarburant et de base pour carburéacteur. Par la conduite 7, on soutire une fraction renfermant généralement, à ce stade, de l'isobutane, du n-butane, du butène-1, des butènes-2, un peu d'isobutène et une quantité mineure de butadiène, et que l'on envoie dans la zone 8 de polymérisation de finition. L'effluent de la zone de polymérisation de finition est acheminé par la conduite 9 dans la zone de fractionnement 10. Par la conduite 13, on recueille un mélange de dimères et de trimères de l'isobutène et du butène-1 et des butènes-2, c'est à dire un supercarburant que l'on mélange dans la conduite 14 avec la coupe supercarburant base pour carburéacteur venant de la conduite 11.

Par la conduite 12, on soutire généralement à ce stade, de l'isobutane, du n-butane, du butène-1, des butènes-2, un tout petit peu d'isobutène et des traces de butadiène, que l'on envoie dans la zone d'hydrogénation sélective 20. L'effluent de la zone d'hydrogénation sélective, est envoyé par la conduite 21 dans la zone 22 de déisobutanisation d'où l'on soutire en tête, par la conduite 23, de l'isobutane et du butène-1 ainsi qu'un peu de butènes-2 et d'isobutène; en fond de colonne, par la conduite 24, on recueille un mélange renfermant essentiellement du butène-1 et des butènes-2. Le mélange de la conduite 24 est fractionné dans la zone de superfractionnement 25. On obtient ainsi, d'une part, par la conduite 26, du butène-1 accompagné de traces d'isobutane et d'isobutène, (la teneur en butène-1 de cette fraction est au moins 99% en poids), et d'autre part, par la conduite 27, un mélange contenant principalement du n-butane, des butènes-2 et un peu de butène-1, le dit mélange étant ensuite mélangé, en premier lieu, avec l'essence provenant par la ligne 34, de la zone dite de polymérisation de finition, et en second lieu avec le polymérisat issu de la zone de polymérisation-dismutation par la ligne 11.

Le mélange subséquent est alors introduit par la conduite 14 dans la zone d'hydrogénation 15 où, en présence d'hydrogène injecté par la conduite 16, le dit mélange subit une hydrogénation totale. Le produit issu de cette zone d'hydrogénation est envoyé par la conduite 17, par exemple, dans une première colonne de fractionnement 18.

En tête de cette colonne, on soutire essentiellement du n-butane qui peut être envoyé, par la conduite 19, à une unité de craquage à la vapeur 28 (par exemple celle d'où provient la coupe $C_4$ traitée conformément à l'invention) où ce n-butane est transformé en grande partie en éthylène, en propène et en essence (ligne 29).

En fond de la colonne 18, on recueille un mélange essence-combustible pour carburéacteur qui est envoyé par la conduite 30 vers une deuxième colonne de fractionnement 31. En tête de cette colonne, on soutire une essence qui peut être envoyée au pool essence par la conduite 32, et en fond de colonne, par la conduite 33, un combustible pour carburéacteur de grandes qualités.

4

**0 068 981**

### Exemple 1 illustrant la figure 1

On se propose de traiter une charge provenant d'un craquage à la vapeur, et de laquelle la majeure partie du butadiène a été extraite. Cette charge à la composition pondérale suivante:

| | |
|---|---|
| — isobutane | 1,3 |
| — n-butane | 6,4 |
| — butène-1 | 27,9 |
| — butènes-2 | 14,5 |
| — isobutène | 49,6 |
| — butadiène | 0,3 |
| | 100 |

Elle est d'abord soumise à un séchage sur un tamis moléculaire 3A (zone 2 de la figure), puis elle est envoyée par la conduite 3 dans une unité 4 de polymérisation-Dismutation, en présence d'un catalyseur qui est une silice-alumine à 85% en poids de silice, à laquelle on a, en outre, ajouté 0,2% en poids de chrôme sous forme de nitrate.

Les conditions opératoires sont les suivantes:

| | |
|---|---|
| pression | 2 MPa |
| température | 50° C |
| Vitesse spatiale VVH | 0,3 |

L'effluent obtenu est soumis à un fractionnement (zone 6 de la figure).

En fond de colonne, par la conduite 11, on recueille un polymérisat qui représente 26,45% en poids par rapport à la charge initiale.

En tête de colonne, par la conduite 7, on soutire une fraction ayant la composition suivante en % poids par rapport à la charge initiale.

| | |
|---|---|
| — isobutane | 2,64 |
| — n-butane | 8,77 |
| — butène-1 | 39,92 |
| — butènes-2 | 20,76 |
| — isobutène | 1,43 |
| — butadiène | 0,03 |
| | 73,55 |

Cette fraction de la conduite 7 est envoyée dans une unité 8 de polymérisation de finition, en présence d'un catalyseur qui est une silice-alumine à 85% en poids de silice, à laquelle on a, en outre, ajouté 0,2% en poids de zinc sous forme de nitrate.

Les conditions opératoires sont les suivantes:

| | |
|---|---|
| pression | 2 MPa |
| température | 80° C |
| Vitesse spatiale VVH | 0,5 |

L'effluent obtenu est ensuite soumis à un fractionnement (zone 10 de la figure).

En fond de colonne, par la conduite 13, on recueille une essence qui représente 3,75% en poids par rapport à la charge initiale. Cette essence est ensuite mélangée avec le polymérisat provenant de la conduite 11.

En tête de la colonne 10, par la conduite 12, on soutire une fraction ayant la composition suivante en poids par rapport à la charge initiale:

| | |
|---|---|
| — isobutane | 3,30 |
| — n-butane | 9,18 |
| — butène-1 | 36,28 |
| — butènes-2 | 20,94 |
| — isobutène | 0,10 |
| — butadiène | 40 ppm |
| | ~ 69,80 |

Cette fraction de la conduite 12 est envoyée dans une unité d'hydrogénation sélective du butadiène 20, en présence d'un catalyseur d'hydrogénation qui est le LD 271 commercialisé par la Société PROCATALYSE.

Les conditions opératoires sont les suivantes:

| pression | 1 MPa |
|---|---|
| température | 40°C |
| Vitesse spatiale VVH | 10 |
| Hydrogène/butadiène | 2 moles/1 mole |

L'effluent obtenu est ensuite envoyé par la conduite 21 dans un déisobutaniseur 22, constitué de deux colonnes en série de 65 plateaux chacune. Par la conduite 23, on recueille une coupe renfermant, en poids par rapport à la charge initiale:

| — isobutane | 3,14 |
|---|---|
| — n-butane | 0 |
| — butène-1 | 1,78 |
| — butènes-2 | 0,46 |
| — isobutène | 0,07 |
| | 5,45 |

Par la conduite 24, on recueille une coupe de composition suivante, en poids par rapport à la charge initiale:

| — isobutane | 0,16 |
|---|---|
| — n-butane | 9,18 |
| — butène-1 | 34,50 |
| — butènes-2 | 20,48 |
| — isobutène | 0,03 |
| — butadiène | 0 |
| | 64,35 |

On fractionne cette coupe dans une zone de superfractionnement 25. La fraction soutirée en tête de colonne par la conduite 26 renferme en poids par rapport à la charge initiale:

| — isobutane | 0,16 |
|---|---|
| — isobutène | 0,03 |
| — butène-1 | 32,84 |
| | 33,03 |

La pureté du butène-1 dans cette coupe est donc de 99,4%.

On recueille au fond de la colonne de superfractionnement 25, une fraction renfermant enpoids par rapport à la charge initiale:

| — n-butane | 9,18 |
|---|---|
| — butène-1 | 1,66 |
| — butènes-2 | 20,48 |
| | 31,32 |

Cette fraction est ensuite mélangée dans les conduites 27 et 13, dans un premier temps avec l'essence provenant, par la ligne 34, de la zone de polymérisation de finition puis dans un deuxième temps avec le polymérisat de la ligne 11 issu de la zone de polymérisation-dismutation 4.

Le mélange subséquent est ensuite dirigé par la conduite 14 vers la zone d'hydrogénation 15 où le produit subit, en présence d'un catalyseur (qui est le LD 265 commercialisé +ar la Société PROCATALYSE), et d'hydrogène apporté par la conduite 16, une hydrogénation totale dans les conditions opératoires suivantes:

| — Pression | 5 MPa |
|---|---|
| — Température d'entrée au réacteur | 30°C |
| — Température de sortie du réacteur | 290°C |
| — Vitesse spatiale VVH | 2 |
| — Hydrogène/Hydrocarbures molaire | 5/1 |

L'effluent issu de la zone d'hydrogénation totale est envoyé par la conduite 17 vers une première colonne de fractionnement 18. En tête de cette première colonne, on soutire par la conduite 19, du n-butane représentant 32,11% en poids par rapport à la charge initiale.

Ce n-butane est recyclé vers la zone de craquage à la vapeur 28. L'effluent en provenance de cette zone de craquage à la vapeur, soutiré par la conduite 29, a la composition suivante, en poids par rapport à la charge initiale

**0 068 981**

| | |
|---|---|
| — hydrogène | 0,74 |
| — méthane | 6,94 |
| — acétylène | 0,06 |
| — éthylène | 13,23 |
| — éthane | 0,29 |
| — propène | 5,27 |
| — propadiène | 0,74 |
| — n-butènes | 0,29 |
| — pentadiène | 0,19 |
| — isopentène | 0,03 |
| — essence de vapocraquage | 4,33 |
| | 32,11 |

En fond de la première colonne de fractionnement 18, on recueille un produit qui est envoyé par la conduite 30 vers une deuxième colonne de fractionnement 31.

En tête de cette deuxième colonne, on soutire par la conduite 32, une essence qui représente 24,73% en poids par rapport à la charge initiale, et dont les caractéristiques principales sont les suivantes:

| | |
|---|---|
| — densité à 15°C | 0,745 |
| — indices d'octane | |
| RON clear | 97 |
| RON éthylé à 0,5‰ | 104 |
| MON clear | 90 |
| MON éthylé à 0,5‰ | 98 |
| — distillation ASTM | |
| PI | 96°C |
| 5% vol | 101°C |
| 10% | 105°C |
| 20% | 112°C |
| 30% | 120°C |
| 40% | 127°C |
| 50% | 141°C |
| 60% | 160°C |
| 70% | 171°C |
| 80% | 177°C |
| 90% | 186°C |
| 95% | 193°C |
| PF | 204°C |
| distillat | 99,5% vol |
| résidu | 0,5% vol |
| pertes | — |

Une telle essence peut être envoyée directement dans un pool essence, car elle répond à toutes les caractéristiques et spécifications concernant les supercarburants.

En fond de cette deuxième colonne de fractionnement 31, on recueille par la conduite 33, un combustible pour carburéacteur représentant 5,88% en poids par rapport à la charge initiale, et dont les caractéristiques principales sont les suivantes:

| | |
|---|---|
| — Point de cristallisation | < −65°C |
| — Point de fumée | 33 mm |

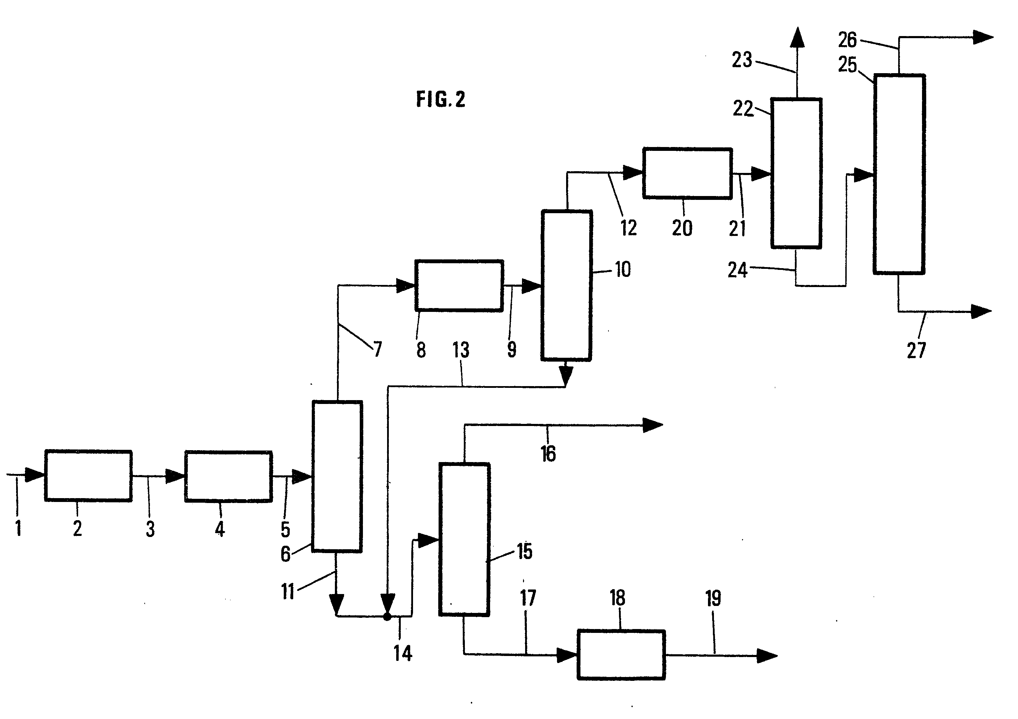

Le procédé selon la figure 2 est caractérisé en ce que:

a)  une coupe $C_4$ oléfinique, qui généralement à ce stade, renferme de l'isobutane, du n-butane, du butène-1, des butènes-2, de l'isobutène et éventuellement du butadiène (moins de 2% et de préférence moins de 0,7% en poids de butadiène), est éventuellement d'abord envoyée dans une zone de séchage (séchage réalisé de façon conventionnelle, par exemple par passage de la coupe sur un tamis moléculaire, de préférence un tamis de type 3A), puis est soumise à une polymérisation-dismutation au cours de laquelle d'une part l'isobutène de la dite coupe est transformé en partie au moins en une essence, et d'autre part l'essence ainsi produite est soumise au moins en partie à une réaction partielle de dismutation de façon à recueillir d'une part une coupe type base pour carburéacteur, et d'autre part des butènes-2 et surtout du butène-1 produits par la dite dismutation,

b)  l'effluent soutiré de la zone de polymérisation-dismutation est soumis à un fractionnement au cours duquel on recueille d'une part un mélange ($\alpha$) d'essence et de base pour carburéacteur, et

7

d'autre part une fraction (β) essentiellement constituée d'isobutane, de n-butane, de butène-1, de butènes-2, d'une légère portion d'isobutène (inférieure à 5% et de préférence à 2% en poids), et d'une mineure quantité de butadiène (inférieure à 2000 parties par million et de préférence à 500 parties par million),

c) la fraction (β) obtenue à l'étape (b) est envoyée dans une zone de polymérisation sélective dite polymérisation de finition, dans laquelle 90% au moins en poids, de l'isobutène résiduel est transformé en essence, en ne transformant en essence qu'une très faible portion de butène-1 et de butènes-2 (inférieure ou égale à 10% en poids),

d) l'effluent soutiré de la zone dite polymérisation de finition effectuée à l'étape (c) est soumis à un fractionnement au cours duquel on recueille d'une part une coupe (γ) constituée en majeure partie d'essence renfermant notamment un mélange de dimères et de trimères de l'isobutène et des butènes-1 et -2, et d'autre part une fraction (δ) essentiellement constituée d'isobutane, de n-butane, de butène-1, de butènes-2, d'une portion mineure d'isobutène (inférieure à 0,3% et de préférence à 0,15% en poids), et de traces de butadiène (inférieures à 100 parties par million),

e) la coupe (δ) issue de l'étape (d) est envoyée dans une zone d'hydrogénation sélective de manière à ce que sa teneur en butadiène soit amenée au maximum à 10 parties par million en poids par rapport au butène-1 de la coupe (δ),

f) l'effluent de l'étape d'hydrogénation sélective (e) est envoyé dans une zone de déisobutanisation en vue d'éliminer la majeure partie de l'isobutane qu'il contenait,

g) la fraction ainsi déisobutanisée est soumise à un fractionnement au cours duquel on recueille, d'une part une coupe renfermant en majeure partie des butènes-2 et du n-butane, et d'autre part une fraction constituée d'au minimum 99% en poids de butène-1; cette coupe butène-1 convenant parfaitement pour être utilisée, notamment, directement en tant que comonomère dans la production de polyéthylène haute densité,

h) le mélange (α) constitué par l'essence et la base pour carburéacteur, issu de l'étape (b), est mélange avec la coupe (γ) d'essence provenant de l'étape (d), le nouveau mélange subséquent étant soumis à un fractionnement au cours duquel on recueille d'une part une coupe essence de haut indice d'octane (supercarburant), qui peut être envoyée à un pool essence, et d'autre part une coupe pouvant servir de base pour carburéacteur (mélange de trimères et tétramères de l'isobutène).

La coupe base pour carburéacteur issue de l'étape (h) est éventuellement envoyée dans une zone d'hydrogénation, à la sortie de laquelle, le produit obtenu constitue un combustible pour carburéacteur dont les qualités sont excellentes.

Au cours de la réaction de polymérisation-dismutation de l'étape (a), l'isobutène est converti essentiellement en une essence constituée essentiellement de dimères et trimères de l'isobutène. La coupe type base pour carburéacteur contient en majeure partie des trimères et tétramères de l'isobutène.

Dans la zone dite de polymérisation de finition, l'isobutène et une petite partie des butènes-1 et -2, sont convertis en dimères et trimères de l'isobutène.

Dans la zone dite de polymérisation-dismutation, les conditions sont telles que l'isobutène réagit, jusqu'à des taux de conversion en essence supérieurs à 95% en poids tandis que, non seulement les butènes-1 et -2 ne sont pas transformés, mais leur concentration se trouve augmentée par la réaction de dismutation d'une partie de l'essence formée à partir de l'isobutène.

Dans la zone dite de polymérisation de finition, les conditions sont telles que l'isobutène réagit jusqu'à des taux de conversion supérieurs à 90% en poids tandis que les conversions globales des butènes normaux (butène-1 et butènes-2 cis et trans), restent inférieures ou égales à 10% en poids et de préférence inférieures à 7%.

Les réactions de polymérisation-dismutation sont effectuées comme indiqués pour la figure 1.

La réaction dite de polymérisation de finition est également effectuée comme indique pour la figure 1.

La réaction d'hydrogénation de la coupe type combustible pour carburéacteur est effectuée comme indiqué pour la figure 1.

## La figure 2 illustre l'invention

La charge oléfinique $C_4$ est introduite par la conduite 1 dans une zone facultative de séchage 2, d'où elle est soutirée par la conduite 3 dans la zone de polymérisation-dismutation 4. L'effluant de la zone de polymérisation-dismutation est acheminé par la conduite 5 dans la zone de fractionnement 6. Par la conduite 11, on recueille essentiellement un mélange de dimères, trimères et tétramères de l'isobutène, c'est-à-dire un mélange de supercarburant et de base pour carburéacteur. Par la conduite 7, on soutire une fraction renfermant généralement, à ce stade, de l'isobutane, du n-butane, du butène-1, des butènes-2, un peu d'isobutène et une quantité mineure de butadiène, et que l'on envoie dans la zone 8 de polymérisation de finition. L'effluent de la zone de polymérisation de finition est acheminé

8

par la conduite 9 dans la zone de fractionnement 10. Par la conduite 13, on recueille un mélange de dimères et de trimères de l'isobutène, du butène-1 et des butènes-2, c'est-à-dire un supercarburant que l'on mélange dans la conduite 14 avec la coupe supercarburant — base pour carburéacteur venant de la conduite 11: le mélange subséquent est introduit par la conduite 14 dans la zone de fractionnement 15. Par la conduite 16, on soutire essentiellement un supercarburant que l'on peut envoyer vers le pool essence. Par la conduite 17, on recueille un mélange de trimères et de tétramères de l'isobutène, c'est-à-dire une base pour carburéacteur qui peut être envoyée vers la zone d'hydrogénation 18. L'effluent de la zone d'hydrogénation 19 constitue un combustible pour carburéacteur de grandes qualités.

Par la conduite 12, on soutire généralement à ce stade, de l'isobutane, du n-butane, du butène-1, des butènes-2, un tout petit peu d'isobutène et des traces de butadiène, que l'on envoie dans la zone d'hydrogénation sélective 20. L'effluent de la zone d'hydrogénation sélective, est envoyé par la conduite 21 dans la zone 22 de déisobutanisation d'où l'on soutire en tête, par la conduite 23, de l'isobutane et du butène-1 ainsi qu'un peu de butènes-2 et d'isobutène; en fond de colonne, par la conduite 24, on recueille un mélange renfermant essentiellement du butène-1 et des butènes-2. Le mélange de la conduite 24 est fractionné dans la zone de superfractionnement 25. On obtient ainsi, d'une part par la conduite 27 des butènes-2, du n-butane et une faible quantité de butène-1, et d'autre part, par la conduite 26, du butène-1 accompagné de traces d'isobutane et d'isobutène. La fraction de la conduite 26 renferme au moins 99% en poids de butène-1. Il s'agit donc là d'une fraction de butène-1 de pureté supérieure à 99%.


Exemple 2 illustrant la figure 2


On se propose de traiter une charge provenant d'un craquage à la vapeur, et de laquelle la majeure partie du butadiène a été extraite. Cette charge a la composition pondérale suivante:

| | |
|---|---|
| — isobutane | 1,3 |
| — n-butane | 6,4 |
| — butène-1 | 27,9 |
| — butènes-2 | 14,5 |
| — isobutène | 49,6 |
| — butadiène | 0,3 |
| | 100 |

Elle est d'abord soumise à un séchage sur un tamis moléculaire 3A (zone 2 de la figure), puis elle est envoyée par la conduite 3 dans une unité 4 de polymérisation-dismutation, en présence d'un catalyseur qui est une silice-alumine à 85% en poids de silice, à laquelle on a, en outre, ajouté du nitrate de chrome, de manière à introduire 0,3% en poids de $Cr_2O_3$.

Les conditions opératoires sont les suivantes:

| | |
|---|---|
| — pression | 2 MPa |
| — température | 50° C |
| — vitesse spatiale VVH | 0,3 |

L'effluent obtenu est soumis à un fractionnement (zone 6 de la figure).

En fond de colonne, par la conduite 11, on recueille un polymérisat qui représente 26,45% en poids par rapport à la charge initiale.

En tête de colonne, par la conduite 7, on soutire une fraction ayant la composition suivante en % poids par rapport à la charge initiale:

| | |
|---|---|
| — isobutane | 2,64 |
| — n-butane | 8,77 |
| — butène-1 | 39,92 |
| — butènes-2 | 20,76 |
| — isobutène | 1,43 |
| — butadiène | 0,03 |
| | 73,55 |

Cette fraction de la conduite 7 est envoyée dans une unité 8 de polymérisation de finition, en présence d'un catalyseur qui est une silice-alumine à 85% en poids de silice, à laquelle on a, en outre, ajouté 0,25% en poids de ZnO sous forme de nitrate de zinc.

Les conditions opératoires sont les suivantes:

| | | |
|---|---|---|
| — | pression | 2 MPa |
| — | température | 50°C |
| — | vitesse spatiale VVH | 0,5 |

L'effluent obtenu est ensuite soumis à un fractionnement (zone 10 de la figure).

En fond de colonne, par la conduite 13, on recueille une essence qui représente 3,75% en poids par rapport à la charge initiale. Cette essence est ensuite mélangée avec le polymérisat provenant de la conduite 11; le mélange subséquent est acheminé par la conduite 14 vers la zone de fractionnement 15.

En tête de colonne, par la conduite 16, on soutire une essence qui représente 24,38% de la charge initiale et qui a les caractéristiques suivantes:

| | | |
|---|---|---|
| — | densité à 15°C | 0,757 |
| — | indices d'octane | |
| | RON clear | 100 |
| | RON éthylé à 0,5‰ | 103,5 |
| | MON clear | 85 |
| | MON éthylé à 0,5‰ | 88 |
| — | distillation ASTM | |
| | PI | 100°C |
| | 5% | 105°C |
| | 10% | 109°C |
| | 20% | 116°C |
| | 30% | 123°C |
| | 40% | 130°C |
| | 50% | 144°C |
| | 60% | 163°C |
| | 70% | 174°C |
| | 80% | 181°C |
| | 90% | 188°C |
| | 95% | 195°C |
| | PF | 206°C |
| | distillat | 99,5% |
| | résidu | 0,5% |
| | pertes | — |

Une telle essence peut être envoyée directement au pool essence, sans passer par une autre étape de fractionnement ou de purification.

En fond de colonne, par la conduite 17, on recueille on polymérisat plus lourd pouvant servir de base pour carburéacteur; ce polymérisat lourd est envoyé dans une zone d'hydrogénation 18, en présence d'un catalyseur d'hydrogénation à 0,3% en poids de palladium sur alumine de surface 70 m$^2$/g.

Les conditions opératoires sont les suivantes:

| | | |
|---|---|---|
| — | pression | 5 MPa |
| — | température | 290°C |
| — | vitesse spatiale VVH | 2 |
| — | hydrogène/hydrocarbures molaire | 5/1 |

L'effluent 19 de la zone d'hydrogénation représente 5,88% en poids de la charge initiale; il constitue un combustible pour carburéacteur de grandes qualités, ses caractéristiques principales sont les suivantes:

| | | |
|---|---|---|
| — | point de cristallisation | $< -65°C$ |
| — | point de fumée | 33 mm |

En tête la colonne 10, par la conduite 12, on soustire une fraction ayant la composition suivante en poids par rapport à la charge initiale:

| | | |
|---|---|---|
| — | isobutane | 3,30 |
| — | n-butane | 9,18 |
| — | butène-1 | 36,28 |
| — | butènes-2 | 20,94 |
| — | isobutène | 0,10 |
| — | butadiène | 40 ppm |
| | | 69,80 |

0 068 981

Cette fraction de la conduite 12 est envoyée dans une unité d'hydrogénation sélective du butadiène 20, en présence d'un catalyseur d'hydrogénation renfermant 0,2% en poids de palladium et 0,2% en poids d'argent sur alumine de surface 15 m$^2$/g.

Les conditions opératoires sont les suivantes:

| | |
|---|---|
| — pression | 1 MPa |
| — température | 40°C |
| — vitesse spatiale VVH | 10 |
| — hydrogène/butadiène | 2 moles/1 mole |

L'effluent obtenu est ensuite envoyé par la conduite 21 dans un déisobutaniseur 22, constitué de deux colonnes en série de 65 plateaux chacune. Par la conduite 23, on recueille une coupe renfermant, en poids par rapport à la charge initiale:

| | |
|---|---|
| — isobutane | 3,14 |
| — n-butane | 0 |
| — butène-1 | 1,78 |
| — butènes-2 | 0,46 |
| — isobutène | 0,07 |
| | 5,45 |

Par la conduite 24, on recueille une coupe de composition suivante, en poids par rapport à la charge initiale:

| | |
|---|---|
| — isobutane | 0,16 |
| — n-butane | 9,18 |
| — butène-1 | 34,50 |
| — butènes-2 | 20,48 |
| — isobutène | 0,03 |
| — butadiène | 0 |

On fractionne cette coupe dans une zone de superfractionnement 25. On recueille en fond de colonne 27, une fraction renfermant en poids par rapport à la charge initiale:

| | |
|---|---|
| — n-butane | 9,18 |
| — butène-1 | 1,66 |
| — butènes-2 | 20,48 |
| | 31,32 |

La fraction soutirée en tête de colonne par la conduite 26 renferme en poids par rapport à la charge initiale:

| | |
|---|---|
| — isobutane | 0,16 |
| — isobutène | 0,03 |
| — butène-1 | 32,84 |
| | 33,03 |

La pureté du butène-1 dans cette coupe est donc de 99,4%.

Dans ce qui précède, on a utilisé, pour hydrogéner le butadiène, un catalyseur renfermant du palladium et de l'argent sur support. Un tel catalyseur présente l'avantage, sur un catalyseur ne renfermant que du palladium sur support, d'éviter l'isomérisation indésirable du butène-1. Ce catalyseur peut renfermer de 0,05 à 0,5% en poids de palladium et de 0,05 à 1% en poids d'argent, le rapport pondéral argent/palladium étant de 0,7 : 1 à 3 : 1, de préférence 1 : 1 à 2,5 : 1. Le support est de préférence l'alumine (surface préférée: 1—100 m$^2$/g) ou la silice (surface préférée: 10—200 m$^2$/g).

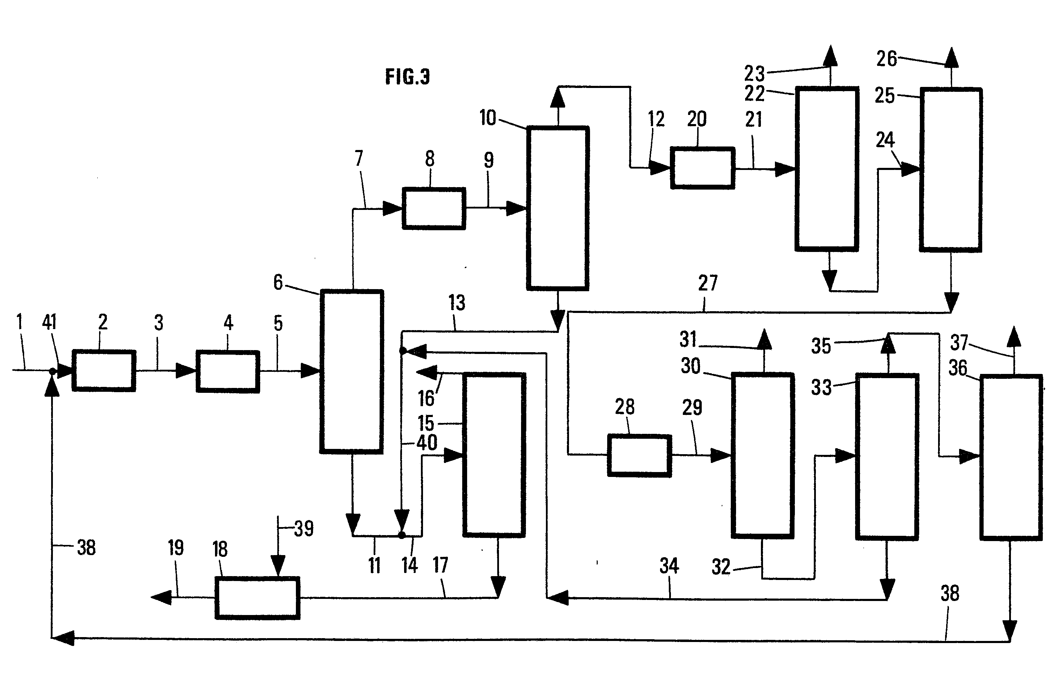

Le procédé selon la figure 3 est un perfectionnement de la figure 2: il est conçu de façon à se que:

a) la charge fraîche, constituée essentiellement d'une coupe C$_4$ oléfinique, qui généralement, à ce stade, renferme de l'isobutane, du n-butane, du butène-1, des butènes-2, de l'isobutène et éventuellement du butadiène (en général moins de 2% et de préférence moins de 0,7% en poids de butadiène), est éventuellement en premier lieu envoyée dans une zone éventuelle de séchage, séchage réalisé de façon conventionnelle, par exemple par passage de la coupe sur un tamis moléculaire, (de préférence un tamis de type 3A). Ensuite, cette charge fraîche est soumise à une polymérisation-dismutation au cours de laquelle d'une part l'isobutène de la dite coupe est transformé en partie au moins en une essence (dimères et trimères de l'isobutène), et d'autre part l'essence ainsi produite est soumise au moins en partie à une réaction partielle de dismutation de

11

## 0 068 981

façon à recueillir une coupe type base pour carburéacteur (mélange de trimères et tétramères de l'isobutène) et des butènes-2 et surtout du butène-1,

b) l'effluent soutiré de la zone de polymérisation-dismutation de l'étape (a), est soumis à un fractionnement au cours duquel on recueille d'une part un mélange ($\alpha$) d'essence et de base pour carburéacteur, et d'autre part une fraction ($\beta$) essentiellement constituée d'isobutane, de n-butane, de butène-1, de butènes-2, d'une légère portion d'isobutène (inférieure à 5% et de préférence à 2% en poids), et d'une mineure quantité de butadiène (inférieure à 2000 parties par millions et de préférence à 500 parties par million),

c) la fraction ($\beta$) obtenue à l'étape (b) est envoyée dans une zone de polymérisation sélective dite polymérisation de finition, dans laquelle 90% au moins en poids de l'isobutène résiduel est transformé en essence, en ne transformant en essence qu'une faible portion de butène-1 et de butènes-2 présents dans la fraction ($\beta$) (inférieure ou égale à 10% en poids),

d) l'effluent soutiré de la zone dite polymérisation de finition effectuée à l'étape (c) est soumis à un fractionnement au cours duquel on recueille d'une part une coupe ($\gamma$) constituée en majeure partie d'essence (ou supercarburant) renfermant notamment un mélange de dimères et de trimères de l'isobutène et des butènes-1 et -2, et d'autre part une fraction ($\delta$) essentiellement constituée d'isobutane, de n-butane, de butène-1, de butènes-2, d'une portion mineure d'isobutène (inférieure à 0,3% et de préférence à 0,15% en poids), et de traces de butadiène (inférieures à 100 parties par million en poids),

e) la coupe ($\delta$) issue de l'étape (d) est ensuite envoyée dans une zone d'hydrogénation dite sélective de manière à ce que sa teneur en butadiène soit amenée au maximum à 10 parties par million en poids par rapport au butène-1 de la coupe ($\delta$),

f) l'effluent de l'étape d'hydrogénation sélective (e), est envoyée dans une zone de déisobutanisation en vue d'éliminer au moins la majeure partie de l'isobutane qu'il contenait,

g) la dite fraction ainsi déisobutanisée est soumise à un fractionnement au cours duquel on recueille, d'une part une coupe ($\varepsilon$) renfermant en majeure partie des butènes-2 et du n-butane, et d'autre part une fraction constituée d'au minimum 99% en poids de butène-1, cette coupe butène-1 convenant parfaitement pour être utilisée, notamment, directement en tant que comonomère dans la production de polyéthylène haute densité, le procédé selon l'invention, étant ensuite caractérisé en ce que:

h) la dite coupe ($\varepsilon$) renfermant en majeure partie des butènes-2 et du n-butane, issue de l'étape (g), est envoyée dans une zone d'isomérisation dans laquelle une partie au moins de ces butènes-2 est isomérisée en phase gazeuse en isobutène et en butène-1, sans toucher à la majeure partie du n-butane, (une faible partie de la coupe ($\varepsilon$) est tranformée par craquage en éthane, éthylène et propène, une autre faible partie est transformée en isobutane et n-butane par une réaction de transfert d'hydrogène, et une autre partie également faible des butènes-2 de la coupe ($\varepsilon$) est tranformée en essence),

i) le produit issu de la zone d'isomérisation de l'étape (h), est envoyé d'abord dans une première colonne de fractionnement, au sommet de laquelle on soutire une coupe contenant principalement de l'éthane, de l'éthylène et du propène et au fond de laquelle on recueille une fraction contenant principalement du butène-1, des butènes-2, de l'isobutène, des traces d'isobutane, du n-butane et une faible proportion d'essence, cette dernière fraction étant alors envoyée ensuite dans une deuxième colonne de fractionnement, au fond de laquelle on recueille la faible fraction d'essence, et au sommet de laquelle on soutire un mélange renfermant du butène-1, de l'isobutène, des butènes-2, et du n-butane, (avec une très faible quantité d'isobutane),

j) le dit mélange obtenu à l'étape précédente étant envoyé dans une colonne de distillation extractive de manière à éliminer en tête la majeure partie de l'isobutane et du n-butane (qui sinon auraient tendance à s'accumuler dans le circuit), la fraction recueillie au fond de la colonne de distillation extractive par solvant (et qui, à ce stade, renferme essentiellement du butène-1, de l'isobutène, des butènes-2 et une faible quantité de n-butane) est recyclée à l'entrée du sécheur éventuel, ou tout au moins à l'entrée de la zone de polymérisation-dismutation, où elle est ainsi mélangée avec la charge fraiche, et

k) le dit mélange ($\alpha$) d'essence et de base pour carburéacteur, issu de l'étape (b), est combiné d'une part avec l'essence obtenue au fond de la deuxième colonne de fractionnement de l'étape (i) et d'autre part avec la coupe ($\alpha$) obtenue à l'étape (d), le mélange subséquent étant envoyé dans une zone de fractionnement, en tête de laquelle on recueille un supercarburant qui peut être envoyé au pool essence, et au fond de laquelle, on recueille un produit qui est éventuellement soumis au moins en partie à une hydrogénation totale, à l'issue de laquelle on récupère un combustible pour carburéacteur dont les qualités sont excellentes.

Les commentaires au sujet des diverses réactions impliquées sont les mêmes que ceux indiquées pour les deux premiers schémas.

En ci qui concerne plus particulièrement la réaction dite d'isomérisation de l'étape h, celle-ci est effectuée en phase gazeuse de préférence en présence de vapeur d'eau, avec un catalyseur disposé en lit fixe ou en lit mobile ou expansé ou en lit fluidisé, à une température d'environ 400 à 600° C sous

12

une pression d'environ 0,01 à 2 MPa (de préférence, la température est d'environ 460 à 530°C, et la pression de 0,1 à 0,5 MPa), avec un débit d'hydrocarbures liquides (vitesse spatiale) d'environ 0,5 à 4 volumes par volume de catalyseur et par heure, le rapport molaire vapeur d'eau/hydrocarbures étant compris entre 0,2 et 2. Dans cette zone d'isomérisation, on convertit, de préférence, au moins 40% (ou mieux 45%) des butènes-2 en isobutène et en butène-1, tandis qu'au moins 80% et de préférence 90% du n-butane ne sont pas touchés.

Pour cette réaction d'isomérisation de l'étape (h), on choisit, de préférence, des catalyseurs fluorés obtenus par exemple par traitement d'une alumine de transition au moyen d'un dérivé du fluor, par exemple l'acide fluorhydrique, le fluorure d'ammonium, l'acide fluorosilicique, l'acide fluoroborique, les composés fluoro organiques, ainsi qu'il est décrit dans la demande de brevet français n° 80/10 783 du 13 mai 1980.

La réaction éventuelle d'hydrogénation de la base type combustible pour carburéacteur (étape k) est effectuée, en présence d'un catalyseur disposé par exemple sous forme d'un lit fixe, le dit catalyseur étant constitué par un métal hydrogénant déposé sur un support sensiblement neutre, par exemple le catalyseur LD 265 commercialisé par la Société PROCATALYSE. La température de réaction est généralement comprise entre 150 et 400°C, sous une pression de 2 à 10 MPa (de préférence la température est d'environ 220 à 300°C et la pression de 4 à 6 MPa), avec un débit d'hydrocarbures liquides (vitesse spatiale) d'environ 1 à 5 volumes par volume de catalyseur et par heure, le rapport molaire Hydrogène/Hydrocarbures à l'entrée du réacteur étant compris entre 2 et 10.

La distillation extractive par solvant de l'isobutane et du n-butane est effectuée en présence de solvants choisis par exemple dans le groupe constitué par l'acétonitrile, le diméthylformamide et la n-méthylpyrrolidone. La température est généralement comprise entre − 10°C et 220°C (de préférence entre 50° et 180°C) et la pression de 0,1 à 1 MPa (de préférence entre 0,3 et 0,7 MPa). Le taux de solvant est plus particulièrement compris entre 1 et 10 en poids (de préférence entre 2 et 6 en poids).

## La figure 3 illustre l'invention

La charge oléfinique $C_4$ est introduite par la conduite 1 dans une zone facultative de séchage 2, d'où elle est soutirée par la conduite 3 dans la zone de polymérisation-dismutation 4. L'effluent de la zone de polymérisation-dismutation est acheminé par la conduite 5 dans la zone de fractionnement 6. Par la conduite 11, on recueille essentiellement un mélange de dimères, trimères et tétramères de l'isobutène, c'est-à-dire un mélange de supercarburant et de base pour carburéacteur. Par la conduite 7, on soutire une fraction renfermant généralement, à ce stade, de l'isobutane, du n-butane, du butène-1, des butènes-2, un peu d'isobutène et une quantité mineure de butadiène, et que l'on envoie dans la zone 8 de polymérisation de finition. L'effluent de la zone de polymérisation de finition est acheminé par la conduite 9 dans la zone de fractionnement 10. Par la conduite 13, on recueille un mélange de dimères et de trimères de l'isobutène, du butène-1 et des butènes-2, c'est-à-dire un supercarburant que l'on mélange par la conduite 40, dans la conduite 14 avec la coupe supercarburant base pour carburéacteur venant de la conduite 11.

Par la conduite 12, on soutire généralement, à ce stade, de l'isobutane, du n-butane, du butène-1, des butènes-2, un tout petit peu d'isobutène et de traces de butadiène, que l'on envoie dans la zone d'hydrogénation sélective 20. L'effluent de la zone d'hydrogénation sélective, est envoyé par la conduite 21 dans la zone 22 de déisobutanisation d'où l'on soutire en tête, par la conduite 23, de l'isobutane et du butène-1 ainsi qu'un peu de butènes-2 et d'isobutène; en fond de colonne, par la conduite 24, on recueille un mélange renfermant essentiellement du butène-1 et des butènes-2. Le mélange de la conduite 24 est fractionné dans la zone de superfractionnement 25. On obtient ainsi, d'une part, par la conduite 26, du butène-1 accompagné de traces d'isobutane et d'isobutène, (la teneur en butène-1 de cette fraction est au moins 99% en poids), et d'autre part, par la conduite 27, un mélange contenant principalement du n-butane, des butènes-2 et une faible quantité de butène-1; ce dernier mélange est ensuite envoyé dans une zone d'isomérisation 28. Le produit issu de la zone d'isomérisation est envoyé par la conduite 29 vers une première colonne de fractionnement 30; en tête de cette colonne, on soutire par la conduite 31 une fraction renfermant essentiellement de l'éthane, de l'éthylène et surtout du propène, cette fraction pouvant être utilisée comme combustible, ou pour un usage pétrochimique; au fond de la colonne 30, on recueille un produit renfermant principalement de l'isobutane, du n-butane, du butène-1, de l'isobutène, des butènes-2 et de l'essence, ce produit étant envoyé par la conduite 32 vers une deuxième colonne de fractionnement 33. En fond de cette colonne, on recueille une essence qui est envoyée par la conduite 34 vers la conduite 13 pour être mélangée dans la conduite 40 avec l'essence provenant, par la conduite 13, de la zone de fractionnement 10, le mélange subséquent étant à son tour mélangé avec le polymérisat de la conduite 11 issu de la zone de fractionnement 6. En tête de la colonne 33, on soutire un produit contenant principalement de l'isobutane, du n-butane, du butène-1, de l'isobutène et des butènes-2; ce produit est envoyé par la conduite 35 vers une zone de distillation extractive 36. En tête de cette zone de distillation extractive, on soutire, par la conduite 37, un produit renfermant principalement de l'isobutane et surtout du n-butane. En fond de la zone de distillation extractive, on recueille une fraction renfermant essentiellement du butène-1, de l'isobutène et des butènes-2, la dite fraction étant ensuite recyclée, par les conduites 38 et 41, à l'entrée de la zone de séchage 2 (ou de la zone 4), où elle est mélangée avec la charge fraiche (A noter qu'en fond de la colonne de distillation extractive, on recueille généralement les butènes et l'isobutène dans le solvant d'extraction. Le solvant est éliminé par distillation simple, par entraînement (strippage) ou par réextraction au moyen d'un solvant auxiliaire non-miscible tel qu'un hydrocarbure paraffinique facilement séparable par distillation des butènes et de l'isobutène).

Les essences des conduites 13 et 34, en mélange dans la conduite 40, et le polymérisat de la conduite 11 sont envoyés ensemble, par la conduite 14, vers une zone de fractionnement 15. En tête de cette colonne, on soutire, essentiellement, par la conduite 16, un supercarburant qui peut être envoyé vers le pool essence. En fond de colonne, on recueille par la conduite 17, un mélange de trimères et de tétramères de l'isobutène, c'est-à-dire une base pour carburéacteur qui peut être dirigé vers une zone d'hydrogénation totale ou partielle 18, l'hydrogène nécessaire étant introduit par la conduite 39; à la sortie de cette zone d'hydrogénation, on recueille, par la conduite 19, un combustible pour carburéacteur de grande qualités.

### Exemple 3 illustrant la figure 3

Le bilan matière dans les diverses sections de l'unité globale est présenté sur le tableau I.
On traite 100 kg d'une charge de composition pondérale suivante:

| | | |
|---|---|---:|
| — | isobutane | 1,3 |
| — | n-butane | 6,4 |
| — | butène-1 | 27,9 |
| — | isobutène | 49,6 |
| — | butènes-2 | 14,5 |
| — | butadiène-1,3 | 0,3 |
| | | 100 |

A l'issue du procédé, l'on obtient les productions nettes suivantes:

| | | |
|---|---|---:|
| — | butène-1 (pureté 99,6) | 42,5 kg |
| — | supercarburant | 31,1 kg |
| — | combustible pour carburéacteur | 7,2 kg |

Tableau I

Bilan matière dans les diverses sections de l'unité

| | Numéros des conduites de la figure | | | | | | | | | | | | | | | |
| | 1 | 3 | 7 | 11 | 12 | 13 | 23 | 26 | 27 | 31 | 34 | 37 | 38 | 16 | 39 | 19 |
|---|---|---|---|---|---|---|---|---|---|---|---|---|---|---|---|---|
| Hydrogène | | | | | | | | | | | | | | | 0,05 | |
| éthane | | | | | | | | | | 0,02 | | | | | | |
| éthylène | | | | | | | | | | 0,10 | | | | | | |
| propène | | | | | | | | | | 0,77 | | | | | | |
| isobutane | 0,90 | 0,90 | 2,03 | | 2,68 | | 2,55 | 0,13 | — | | | 0,06 | | | | |
| n-butane | 4,42 | 5,04 | 7,07 | | 7,47 | | — | — | 7,47 | | | 7,06 | 0,62 | | | |
| butène-1 | 19,28 | 26,05 | 33,84 | | 32,30 | | 1,58 | 29,25 | 1,47 | 0,09 | | 0,06 | 6,77 | | | |
| isobutène | 34,27 | 41,92 | 1,22 | | 0,10 | | 0,07 | 0,03 | — | 28 ppm | | 0,07 | 7,65 | | | |
| butènes-2 | 10,03 | 25,87 | 33,43 | | 31,40 | | 0,69 | | 30,71 | | | 0,15 | 15,84 | | | |
| butadiène 1-3 | 0,21 | 0,22 | 0,02 | | 30 ppm | | — | — | — | | | | 0,01 | | | |
| polymérisat | | | | 22,39 | | | | | | | | | | | | |
| essence | | | | | | 3,66 | | | | | 0,38 | | | 21,50 | | |
| combustible pour carburéacteur | | | | | | | | | | | | | | | | 4,98 |
| Total | 69,11 | 100 | 77,61 | 22,39 | 73,95 | 3,66 | 4,89 | 29,41 | 39,65 | 0,98 | 0,38 | 7,40 | 30,89 | 21,50 | 0,05 | 4,98 |

La pureté du butène-1 est de 99,5%; il peut donc être utilisé comme comonomère dans la production de polyéthylène haute densité.

L'indice d'octane du supercarburant est le suivant:

RON clear = 100

Le combustible pour carburéacteur est de très bonne qualité:

| | |
|---|---|
| point de cristallisation | $< -65''$C |
| point de fumée | 33 mm |

Les conditions opératoires dans cet exemple sont les suivantes:

La charge est séchée (zone 2) sur un tamis moléculaire 3A.

La zone de polymérisation-dismutation 4 fonctionne à 50°C, sous 2 MPa, avec une VVH de 0,3, en présence d'un catalyseur qui est une silice alumine à 85% en poids de silice, à laquelle on a, en outre, ajouté du nitrate de chrome, de manière à introduire dans le catalyseur 0,3% en poids de $Cr_2O_3$. La zone de polymérisation de finition 8 fonctionne à 50°C, sous 2 MPa, avec une VVH de 0,5 en présence d'un catalyseur qui est une silice alumine à 85% en poids de silice, à laquelle on a ajouté en outre 0,25% en poids d'oxyde de zinc sous forme de nitrate de zinc. La zone d'hydrogénation 18 fonctionne à 290°C, sous 5 MPa, avec une VVH de 2 et un rapport molaire hydrogène/hydrocarbures égal à 5/1 en présence d'un catalyseur d'hydrogénation à 0,3% en poids de palladium sur une alumine de surface 70 $m^2$/g. La zone d'hydrogénation sélective 20 fonctionne à 40°C, sous 1 MPa, avec une VVH de 10 et un rapport hydrogène/butadiène égal à 2 moles/1 mole en présence d'un catalyseur qui est une alumine de surface 15 $m^2$/g renfermant 0,2% en poids de palladium et 0,2% en poids d'argent. Le déisobutaniseur 22 est constitué de deux colonnes en série de 65 plateaux chacune. L'isomérisation dans la zone 28 est effectuée, en présence de vapeur d'eau, à 450°C sous 0,1 MPa, avec une VVH égale à 2 et un rapport molaire $H_2O$/HC égal à 1,30 et en présence d'un catalyseur utilisé sous forme de billes d'alumine (de type $\gamma$ cubique) de 1 à 2 mm de diamètre, possédant les caractéristiques suivantes:

| | |
|---|---|
| densité | 1,23 |
| densité structurale | 3,27 |
| volume poreux total | 0,57 $cm^3$/g |
| surface spécifique | 200 $m^2$/g |

et ayant la composition suivante:

teneur en métaux alcalins inférieure à 100 ppm (poids)
teneur en métaux alcalino-terreux inférieure à 300 ppm (poids)
teneur en poids en fluor: 1%

La colonne de distillation extractive 36 comporte plateaux. Le solvant utilisé est le diméthylformamide qui est envoyé dans la colonne à 8 étages du sommet, avec un débit égal à 4,2 fois celui de l'alimentation. Les vapeurs de têtes sont condensées et la moitié du condensat est recyclée en tête de colonne, en tant que reflux, tandis que l'autre moitié du condensat est évacuée du système.

## Revendications

1. Procédé d'obtention du butène-1 de haute pureté et de supercarburant à partir d'une coupe $C_4$ oléfinique caractérisé en ce que:

a) une coupe $C_4$ oléfinique, qui à ce stade renferme de l'isobutane, du n-butane, du butène-1, des butènes-2, de l'isobutène et moins de 2% de butadiène en poids, est soumise à une polymérisation-dismutation en phase liquide, en présence d'au moins un catalyseur acide, (à base d'au moins un composé choisi dans le groupe constitué par les silice-alumines, les silice-magnésies, les bores-alumines, l'acide phosphorique sur kieselguhr ou sur silice ou sur quartz et parmi des catalyseurs du type acide phosphorique solide et les alumines de transition traitées par au moins un dérivé acide du fluor) entre 10 et 120°C, sous une pression de 0,3 à 10 MPa, avec un débit d'hydrocarbures liquides d'environ 0,05 à 2 volumes par volume de catalyseur et par heure, au cours de laquelle d'une part, les butènes-1 et -2 ne sont sensiblement pas transformés, et l'isobutène de ladite coupe est transformé en partie au moins en une essence, et d'autre part l'essence ainsi produite est soumise au moins en partie à une réaction partielle de dismutation de façon à former une coupe type base pour carburéacteur et des butènes-2 et surtout du butène-1 produits par ladite dismutation,

b) l'effluent soutiré de la zone de polymérisation-dismutation est soumis à un fractionnement au cours duquel on recueille d'une part un mélange ($\alpha$) d'essence et de base pour carburéacteur, et d'autre part une fraction ($\beta$) essentiellement constituée d'isobutane, de n-butane, de butène-1, de butènes-2, de moins de 5% en poids d'isobutène, et de moins de 2000 ppm de butadiène,

c) la coupe ($\beta$) obtenue à l'étape (b) est envoyée dans une zone de polymérisation sélective dite polymérisation de finition, dans laquelle plus de 90% de l'isobutène résiduel est transformé en essence, tandis que les conversions globales des butènes normaux restent inférieures ou égales à 10%, cette réaction de polymérisation de finition étant effectuée en phase liquide, en présence d'au moins un catalyseur acide défini comme le catalyseur de la zone de polymérisation-dismutation, à une température d'environ 30 à 200°C, sous une pression d'environ 0,5 à 20 MPa, avec un débit d'hydrocarbures liquides (vitesse spatiale) d'environ 0,2 à 4 volumes par volume de catalyseur et par heure,

d) l'effluent soutiré de la zone dite polymérisation de finition effectuée à l'étape (c) est soumis à un fractionnement au cours duquel on recueille d'une part une coupe ($\gamma$) constituée en majeure partie d'essence renfermant notamment un mélange de dimères et de trimères de l'isobutène et des butènes-1 et -2, et d'autre part une fraction ($\delta$) essentiellement constituée d'isobutane, de n-butane, de butène-1, de butènes-2, de moins de 0,3% en poids d'isobutène et moins de 100 ppm de butadiène,

e) la coupe ($\delta$) issue de l'étape (d) est envoyée dans une zone catalytique d'hydrogénation sélective fonctionnant à une température de réaction comprise entre 10 et 100°C, sous une pression de 0,1 à 3 MPa, avec un débit d'hydrocarbures liquides d'environ 2 à 20 volumes par volume de catalyseur et par heure, le rapport molaire Hydrogène/butadiène, à l'entrée du réacteur, étant compris entre 0,5 et 5, de manière à ce que la teneur en butadiène de ladite coupe ($\delta$) soit amenée au maximum à 10 parties par million en poids par rapport au butène-1 de la coupe ($\delta$),

f) l'effluent issu de l'étape d'hydrogénation sélective (e), est envoyé dans une zone de déisobutanisation en vue d'éliminer la majeure partie de l'isobutane qu'il contenait,

g) ladite fraction ainsi déisobutanisée est soumise à un fractionnement au cours duquel on recueille, d'une part une coupe renfermant en majeure partie des butènes-2 et du n-butane, et d'autre part une fraction constituée d'au minimum 99% en poids de butène-1, et en ce que le mélange ($\alpha$) constitué par l'essence et la base pour carburéacteur issue de l'étape (b), est mélangé avec la coupe ($\gamma$) d'essence provenant de l'étape (d).

2. Procédé selon la revendication 1 d'obtention du butène-1 de haute pureté et de supercarburant à partir d'une coupe $C_4$ oléfinique, caractérise en ce que (voir figure 1):

a) une coupe $C_4$ oléfinique renfermant de l'isobutane, du n-butane, du butène-1, des butènes-2, de l'isobutène et moins de 2% en poids de butadiène, est soumise à une polymérisation-dismutation dans une zone (4), effectuée en phase liquide, au cours de laquelle au moins 95% en poids de l'isobutène réagissent en se transformant en une essence essentiellement constituée de dimères et trimères de l'isobutène, une partie de cette essence se transformant d'une part en butènes-2 et butène-1 et d'autre part en une coupe type base pour carburéacteur à base de trimères et tétramères de l'isobutène,

b) l'effluent soutiré de la zone de polymérisation-dismutation (4) est soumis à un fractionnement dans la zone (6) au cours duquel on recueille d'une part un mélange ($\alpha$) d'essence et de base pour carburéacteur et d'autre part une fraction ($\beta$) essentiellement constituée d'isobutane, de n-butane, de butène-1, de butènes-2, de moins de 5% en poids d'isobutène et de moins de 2000 ppm de butadiène,

c) la fraction ($\beta$) obtenue à l'étape (b) est envoyée dans une zone(8) de polymérisation sélective dite de finition, dans laquelle 90% au moins, en poids, de l'isobutène qu'elle renferme sont transformés en essence, tandis que les conversions globales des butènes-2 et butène-1 restent inférieures ou égales à 10% en poids,

d) l'effluent de la zone de polymérisation de finition est soumis à un fractionnement [zone (10)] au cours duquel on recueille d'une part une coupe ($\gamma$) constituée en majeure partie d'essence, renfermant des dimères et des trimères de l'isobutène et des butènes-1 et -2, et d'autre part une fraction ($\delta$) essentiellement constituée d'isobutane, de n-butane, de butène-1, de butènes-2, de moins de 0,3% en poids d'isobutène et de moins de 100 ppm de butadiène,

e) la coupe ($\delta$) issue de l'étape (d) est envoyée dans une zone (20) d'hydrogénation sélective de manière à réduire sa teneur en butadiène à moins de 10 parties par million par rapport au butène-1 présent dans la coupe ($\delta$),

f) l'effluent de l'étape (e) est envoyé dans une zone (22) de déisobutanisation en vue d'éliminer la majeure partie de l'isobutane qu'il contenait,

g) la fraction ainsi déisobutanisée est soumise à un fractionnement [zone (25)] au cours duquel on recueille, d'une part, une coupe renfermant en majeure partie des butènes-2 et du n-butane et d'autre part une fraction constituée d'au moins 99% en poids de butène-1, et

h) le mélange ($\alpha$) constitué par l'essence et la base pour carburéacteur, issu de l'étape (b), est mélangé avec la coupe ($\gamma$) d'essence provenant de l'étape (d) et avec la coupe $C_4$ riche en

butènes-2 et en n-butane en provenance de l'étape (g), le nouveau mélange subséquent étant soumis à une hydrogénation dans la zone (15),

i)  l'effluent d'hydrogénation de l'étape (h) étant soumis à au moins un fractionnement au cours duquel on recueille du n-butane, une coupe essence et une coupe combustible.

3. Procédé selon la revendication 2 dans lequel en outre ladite coupe ($\gamma$) renferme, en poids, moins de 2% d'isobutène et moins de 500 ppm de butadiène et dans lequel ladite coupe ($\delta$) renferme moins de 0,15% en poids d'isobutène.

4. Procédé selon l'une des revendications 2 et 3 dans lequel la coupe n-butane recueillie au cours du fractionnement réalisé à l'étape (i) est envoyé dans une zone de craquage à la vapeur.

5. Procédé selon l'une des revendications 2 à 4 dans lequel la coupe $C_4$ oléfinique, traitée conformément à l'invention, provient d'une zone de craquage à la vapeur et dans lequel la coupe n-butane recueillie au cours du fractionnement réalisé à l'étape (i) est recyclée dans ladite zone de craquage à la vapeur.

6. Procédé selon la revendication 1 d'obtention de butène-1 de haute pureté à partir d'une coupe $C_4$ oléfinique, caractérisé en ce que (voir figure 2):

a)  une coupe $C_4$ oléfinique renfermant de l'isobutane, du n-butane, du butène-1, des butènes-2, de l'isobutène et moins de 2% en poids de butadiène, est soumise à une polymérisation-dismutation, dans une zone (4), effectuée en phase liquide, au cours de laquelle au moins 95% en poids de l'isobutène réagissent en se transformant en une essence essentiellement constituée de dimères et trimères de l'isobutène, une partie de cette essence se transformant d'une part en butènes-2 et butène-1 et d'autre part en une coupe type base pour carburéacteur à base de trimères et tétramères de l'isobutène,

b)  l'effluent soutiré de la zone de polymérisation-dismutation (4) est soumis à un fractionnement dans la zone (6) au cours duquel on recueille d'une part un mélange ($\alpha$) d'essence et de base pour carburéacteur et d'autre part une fraction ($\beta$) essentiellement constituée d'isobutane, de n-butane, de butène-1, de butènes-2, de moins de 5% en poids d'isobutène et de moins de 2000 ppm de butadiène,

c)  la fraction ($\beta$) obtenue à l'étape (b) est envoyée dans une zone (8) de polymérisation sélective dite de finition, dans laquelle 90% au moins, en poids, de l'isobutène qu'elle renferme est transformé en essence, tandis que les conversions globales des butènes-2 et butène-1 restent inférieures ou égales à 10% en poids,

d)  l'effluent de la zone de polymérisation de finition est soumis à un fractionnement [zone (10)] au cours duquel on recueille d'une part une fraction ($\gamma$) constituée en majeure partie d'essence, renfermant des dimères et des trimères de l'isobutène et des butènes-1 et -2, et d'autre part une fraction ($\delta$) essentiellement constituée d'isobutane, de n-butane, de butène-1, de butènes-2, de moins de 0,3% en poids d'isobutène et de moins de 100 ppm de butadiène,

e)  la fraction ($\delta$) issue l'étape (d) est envoyée dans une zone (20) d'hydrogénation sélective de manière à réduire sa teneur en butadiène à moins de 10 parties par million par rapport au butène-1 présent dans la fraction ($\delta$),

f)  l'effluent de l'étape (e) est envoyé dans une zone (22) de désisobutanisation en vue d'éliminer la majeure partie de l'isobutane qu'il contenait,

g)  la fraction ainsi désisobutanisée est soumise dans la zone (25), à un fractionnement au cours duquel on recueille, d'une part, une coupe renfermant en majeure partie des butènes-2 et du n-butane et d'autre part une fraction constitué d'au moins 99% en poids de butène-1, et

h)  le mélange ($\alpha$) constitué par l'essence et la base pour carburéacteur issu de l'étape (b), est mélange avec la fraction ($\gamma$) d'essence provenant de l'étape (d), le nouveau mélange subséquent étant soumis, dans la zone 15, à un fractionnement au cours duquel on recueille d'une part une coupe essence de haut indice d'octane et d'autre part une coupe pour carburéacteur.

7. Procédé selon la revendication 6 dans lequel en outre ladite fraction ($\beta$) renferme, en poids, moins de 2% d'isobutène et moins de 500 ppm de butadiène et dans lequel ladite fraction ($\delta$) renferme moins de 0,15% en poids d'isobutène.

8. Procédé selon la revendication 6 dans lequel en outre ladite coupe utilisable comme carburéacteur, obtenue à l'issue du fractionnement de l'étape (h), est envoyée dans une zone d'hydrogénation avant l'être utilisée comme combustible.

9. Procédé selon l'une quelconque des revendications 6 à 8, dans lequel l'hydrogénation sélective de l'étape (e) est effectuée en présence d'un catalyseur supporté renfermant 0,05 à 0,5% en poids de palladium et 0,05 à 1% en poids d'argent, le rapport pondéral argent/palladium étant de 0,7 : 1 à 3 : 1, le support du catalyseur étant une alumine ayant une surface de 1 à 100 m²/g.

10. Procédé selon l'une quelconque des revendications 6 à 9, dans lequel la polymérisation-dismutation de l'étape (a) et la polymérisation de finition de l'étape (c) sont effectuées en présence d'un catalyseur de silice-alumine.

11. Procédé selon la revendication 10, dans lequel le catalyseur de polymérisation-dismutation

18

renferme en outre 0,1 à 5% en poids d'oxyde de chrome et le catalyseur de polymérisation de finition renferme en oute 0,1 à 5% en poids d'oxyde de zinc.

12. Procédé selon la revendication 1 conjointe de butène-1 de haute pureté, de supercarburant et de combustible pour carburéacteur, à partir d'une coupe C$_4$ oléfinique, et qui consiste en ce que (voir figure 3):

a) une charge fraiche, constituée essentiellement d'une coupe C$_4$ oléfinique renfermant de l'isobutane, du n-butane, du butène-1, des butènes-2, de l'isobutène et moins de 2% en poids de butadiène, est soumise à une polymérisation-dismutation, dans une zone (4), effectuée en phase liquide, au cours de laquelle d'une part l'isobutène de ladite coupe est essentiellement transformé au moins en partie en une essence (dimères et trimères de l'isobutène) et d'autre part l'essence ainsi produite est soumise au moins en partie à une réaction partielle de dismutation de façon à recueillir une coupe base pour carburéacteur (mélange de trimères et tétramères de l'isobutène) et des butènes-2 et surtout du butène-1,

b) l'effluent soutiré de la zone de polymérisation-dismutation (4) est soumis à un fractionnement, dans la zone (6) au cours duquel on recueille d'une part un mélange ($\alpha$) d'essence et de base pour carburéacteur et d'autre part une fraction ($\beta$) essentiellement constituée d'isobutane, de n-butane, de butène-1, de butènes-2, de moins de 5% en poids d'isobutène et de moins de 2000 ppm en poids de butadiène,

c) la fraction ($\beta$) obtenue à l'étape (b) est envoyée dans une zone (8) de polymérisation sélective dite polymérisation de finition, dans laquelle 90% au moins, en poids, de l'isobutène qu'elle renferme sont transformés en essence, la réaction de polymérisation sélective étant effectuée en phase liquide, les conversions globales des butènes-2 et du butène-1 restant inférieures ou égales à 10% en poids,

d) l'effluent de la zone de polymérisation de finition est soumis à un fractionnement [zone (10)] au cours duquel on recueille d'une part une fraction ($\gamma$) constituée en majeure partie d'essence, renfermant notamment des dimères et des trimères de l'isobutène et des butènes-1 et -2, et d'autre part une fraction ($\delta$) essentiellement constituée d'isobutane, de n-butane, de butène-1, de butènes-2, de moins de 0,3% en poids d'isobutène et de moins de 100 ppm en poids de butadiène,

e) la fraction ($\delta$) issue de l'étape (d) est envoyée dans une zone (20) d'hydrogénation sélective de manière à réduire sa teneur en butadiène à moins de 10 parties par million en poids par rapport au butène-1 présent dans la fraction ($\delta$),

f) l'effluent de l'étape (e) est envoyé dans une zone (22) de déisobutanisation en vue l'éliminer au moins la majeure partie de l'isobutane qu'il contenait,

g) la fraction ainsi déisobutanisée est soumise à un fractionnement, dans la zone (25), au cours duquel on recueille, d'une part, une coupe ($\varepsilon$) renfermant en majeure partie des butènes-2 et du n-butane et d'autre part une fraction constituée d'au moins 99% en poids de butène-1, le procédé étant caractérisé en ce que, ensuite,

h) ladite coupe ($\varepsilon$) renfermant en majeure partie des butènes-2 et du n-butane, issue de l'étape (g) est envoyée par une conduite (27), dans une zone d'isomérisation (28) dans laquelle une partie au moins de ces butènes-2 est isomérisée en phase gazeuse en isobutène et en butène-1, sans toucher à la majeure partie du n-butane,

i) le produit issu de la zone d'isomérisation de l'étape (h) est envoyé d'abord dans une zone (30) de fractionnement, au fond de laquelle on recueille une fraction contenant principalement du butène-1, des butènes-2, de l'isobutène, de l'isobutane, du n-butane et de l'essence, cette fraction étant envoyée dans une zone (33) de fractionnement au fond de laquelle on recueille l'essence et au sommet de laquelle on soutire un mélange renfermant notamment de l'isobutène, du butène-1, des butènes-2, et de l'isobutane,

j) ledit mélange obtenu à l'étape précédente, au sommet de la zone (33) de fractionnement, est envoyé dans une zone (36) de distillation extractive par solvant de façon à recueillir un mélange renfermant notamment de l'isobutène, du butène-1 et des butènes-2 que l'on recycle vers l'entrée de la zone (4) de polymérisation-dismutation et

k) le mélange ($\alpha$) d'essence et de base pour carburéacteur, issu de l'étape (b), est combiné d'une part avec l'essence obtenue en fond de la zone (33) de fractionnement de l'étape (i) et d'autre part avec la fraction ($\gamma$) obtenue à l'étape (d), le mélange subséquent étant envoyé dans une zone (15) de fractionnement en tête de laquelle on recueille un supercarburant et en fond de laquelle, on recueille un combustible pour carburéacteur.

13. Procédé selon la revendication 12 dans lequel le combustible pour carburéacteur obtenu à l'étape (k) est soumis ensuite à une hydrogénation au cours de laquelle ladite partie des butènes-2 est isomérisée en présence de vapeur d'eau, le rapport molaire vapeur d'eau/hydrocarbures étant compris entre 0,2 et 2.

14. Procédé selon la revendication 13 dans lequel on isomérise les butènes-2 en présence d'un catalyseur à base d'une alumine fluorée.

15. Procédé selon l'une des revendications 13 et 14 dans lequel, au cours de l'isomérisation, on convertit au moins 40%, et de préférence au mons 45%, des butènes-2 en isobutène et en butène-1, tandis que 80% au moins, et de préférence 90% au moins du n-butane ne sont pas touchés.

**Patentansprüche**

1. Verfahren zur Herstellung von Buten-1 hoher Reinheit und von Superkraftstoff, ausgehend von einem olefinischen $C_4$-Schnitt, dadurch gekennzeichnet, daß

a) ein olefinischer $C_4$-Schnitt, der in diesem Stadium Isobutan, n-Butan, Buten-1, Butene-2, Isobuten und weniger als 2 Gew.-% Butadien enthält, einer Polymerisation-Disproportionierung in flüssiger Phase in Gegenwart von zumindest einem sauren Katalysator (auf der Basis von zumindest einer Verbindung aus der Gruppe der Siliziumdioxid-Aluminiumoxide, der Siliziumdioxid-Magnesiumoxide, der Boroxid-Aluminiumoxide, Phosphorsäure auf Kieselgur oder auf Siliziumdioxid oder auf Quarz und aus den Katalysatoren des Typs feste Phosphorsäure und der Übergangsaluminiumoxide, behandelt mit zumindest einem sauren Derivat von Fluor) zwischen 10 und 120° C unter einem Druck von 0,3 bis 10 MPa mit einem Durchsatz an flüssigem Kohlenwasserstoff von etwa 0,05 bis 2 Volumen pro Volumen Katalysator pro Stunde unterworfen wird, im Verlauf welcher einerseits die Butene-1 und -2 nicht wesentlich umgewandelt werden und das Isobuten dieses Schnittes zumindest teilweise in einen Treibstoff überführt wird und andererseits der so erzeugte Treibstoff zumindest teilweise einer partiellen Disproportionierungsreaktion derart unterworfen wird, daß man einen Schnitt vom Typ Düsentreibstoffbasis und Butene-2 und vor allem Buten-1, die durch diese Disproportionierung erzeugt sind, bildet;

b) der Abfluß, der aus der Polymerisations-Disproportionierungszone abgezogen ist, einer Fraktionierung unterworfen wird, im Verlaufe welcher man einerseits ein Gemisch ($\alpha$) von Treibstoff und der Düsentreibstoffbasis und andererseits eine Fraktion ($\beta$) gewinnt, die im wesentlichen aus Isobutan, n-Butan, Buten-1, den Butenen-2, weniger als 5 Gew.-% Isobuten und weniger als 2000 ppm Butadien besteht;

c) der Schnitt ($\beta$) der in der Stufe (b) erhalten ist, in eine Zone der selektiven Polymerisation geführt wird, die Fertigstellungspolymerisation genannt wird, worin mehr als 90% des restlichen Isobutens in Treibstoff überführt wird, während die Gesamtumwandlung der Normalbutene unter oder gleich 10% beträgt, wobei diese Reaktion der Fertigstellungspolymerisation in flüssiger Phase in Gegenwart von zumindest einem sauren Katalysator, wie er für den Katalysator der Polymerisations-Disproportionierungszone definiert ist, bei einer Temperatur von etwa 30 bis 200° C unter einem Druck von etwa 0,5 bis 20 MPa mit einem flüssigen Kohlenwasserstoffdurchsatz (Raumgeschwindigkeit) von etwa 0,2 bis 4 Volumen pro Volumen Katalysator pro Stunde durchgeführt wird;

d) der Abstrom, der aus dieser Fertigstellungspolymerisation, die in Stufe (c) bewirkt wird, abgezogen wird, einer Fraktionierung unterworfen wird, im Verlaufe welcher man einerseits einen Schnitt ($\gamma$) gewinnt, der zum größeren Teil aus Treibstoff besteht, der insbesondere ein Gemisch von Dimeren und Trimeren des Isobutens und der Butene-1 und -2 enthält, und andererseits eine Fraktion ($\delta$), die im wesentlichen aus Isobutan, n-Butan, Buten-1 und den Butenen-2, weniger als 0,3 Gew.-% Isobuten und weniger als 100 ppm Butadien besteht;

e) der Schnitt ($\delta$), der aus der Stufe (d) stammt, in eine katalytische Zone der selektiven Hydrierung geführt wird, die bei einer Reaktionstemperatur zwischen 10 und 100° C, unter einem Druck von 0,1 bis 3 MPa mit einem Durchsatz an flüssigem Kohlenwasserstoff von etwa 2 bis 20 Volumen pro Volumen Katalysator und pro Stunde, wobei das molare Verhältnis Wasserstoff/Butadien am Eingang des Reaktors zwischen 0,5 und 5 liegt, derart arbeitet, daß der Gehalt an Butadien dieses Schnittes ($\delta$) auf höchstens 10 Gew.-Teile pro Million, bezogen auf Buten-1, des Schnittes ($\delta$) gebracht wird;

f) der aus der selektiven Hydrierungsstufe (e) austretende Abfluß in eine Zone der Deisobutanisierung gebracht wird, um den größeren Teil des Isobutans zu entfernen, den er enthält;

g) diese so deisobutanisierte Fraktion einer Fraktionierung unterworfen wird, im Verlaufe welcher man einerseits einen Schnitt gewinnt, der zum größeren Teil Butene-2 und n-Butan enthält, und andererseits eine Fraktion, die aus mindestens 99 Gew.-% Buten-1 besteht und wobei die Mischung ($\alpha$), die aus Treibstoff und aus der Düsentreibstoffbasis aus der Stufe (b) besteht, mit dem Schnitt ($\gamma$) an Treibstoff, der aus der Stufe (d) stammt, gemischt wird.

2. Verfahren nach Anspruch 1 zur Erzielung von Buten-1 hoher Reinheit und von Supertreibstoff ausgehend von einem olefinischen $C_4$-Schnitt, dadurch gekennzeichnet, daß (siehe Figur 1):

a) ein olefinischer $C_4$-Schnitt, der Isobutan, n-Butan, Buten-1, die Butene-2, Isobuten und weniger als 2 Gew.-% Butadien enthält, einer Polymerisation-Disproportionierung in einer Zone (4) unterworfen wird, die in flüssiger Phase durchgeführt wird und im Verlaufe welcher zumindest 95 Gew.-% des Isobutens reagieren und sich in einen Treibstoff umwandeln, der im wesentlichen aus Dimeren und Trimeren des Isobutens besteht, ein Teil dieses Treibstoffes sich einerseits in Butene-2 und Buten-1 und andererseits in einen Schnitt vom Typ Düsentreibstoffbasis auf der Basis der Trimeren und Tetrameren des Isobutens umwandelt;

b) der aus der Polymerisations-Disproportionierungszone (4) abgezogene Abstrom einer Fraktionierung in der Zone (6) unterworfen wird, im Verlaufe welcher man einerseits ein Gemisch ($\alpha$) von Treibstoff und Düsentreibstoffbasis und andererseits eine Fraktion ($\beta$) gewinnt, die im wesentlichen aus Isobutan, n-Butan, Buten-1 und Butenen-2, weniger als 5 Gew.-% Isobuten und weniger als 2000 ppm Butadien besteht;

c) die in der Stufe (b) erhaltene Fraktion ($\beta$) in eine Zone (8) der selektiven Polymerisation, die Fertigstellungspolymerisation genannt wird, führt, worin zumindest 90 Gew.-% des Isobutens, das sie enthält, in Treibstoff überführt werden, während die Gesamtumwandlung der Butene-2 und des Buten-1 unter oder gleich 10 Gew.-% bleibt;

d) der Abstrom der Zone der Fertigstellungspolymerisation einer Fraktionierung [Zone (10)] unterworfen wird, im Verlaufe welcher man einerseits einen Schnitt ($\gamma$) gewinnt, der zum größeren Teil aus Treibstoff besteht, welcher die Dimeren und die Trimeren von Isobuten und der Butene-1 und -2 enthält, und andererseits eine Fraktion ($\delta$), die im wesentlichen aus Isobutan, n-Butan, Buten-1 und den Butenen-2, weniger als 0,3 Gew.-% Isobuten und weniger als 100 ppm Butadien besteht;

e) der Schnitt ($\delta$), der aus der Stufe (d) stammt, in eine solche Zone (20) zur selektiven Hydrierung geführt wird, daß sein Gehalt an Butadien auf weniger als 10 Teile pro Million, bezogen auf im Schnitt ($\delta$) vorhandenes Buten-1, vermindert wird;

f) der Abstrom der Stufe (e) in eine Zone (22) zur Deisobutanisierung geführt wird, um den größeren Teil des Isobutans, den er enthält, zu beseitigen;

g) die so deisobutanisierte Fraktion einer Fraktionierung [Zone (25)] unterworfen wird, im Verlaufe welcher man einerseits einen Schnitt gewinnt, der zum hauptsächlichen Teil Butene-2 und n-Butan enthält, und andererseits eine Fraktion, die zumindest 99 Gew.-% aus Buten-1 besteht, und

h) die Mischung ($\alpha$), die aus dem Treibstoff und der Düsentreibstoffbasis besteht, und aus der Stufe (b) stammt, mit dem Schnitt ($\gamma$) an Treibstoff gemischt wird, der aus der Stufe (d) stammt und mit dem C$_4$-Schnitt, der reich an Butenen-2 und an n-Butan ist und aus der Stufe (g) stammt, wobei die neue Mischung dann einer Hydrierung in der Zone (15) unterworfen wird;

i) der Abstrom der Hydrierung aus der Stufe (h) zumindest einer Fraktionierung unterworfen wird, im Verlaufe welcher man n-Butan, einen Treibstoffschnitt und einen Brennstoffschnitt gewinnt.

3. Verfahren nach Anspruch 2, dadurch gekennzeichnet, daß der Schnitt ($\beta$) außerdem weniger als 2% Isobuten und weniger als 500 ppm Butadien enthält und daß der Schnitt ($\delta$) weniger als 0,15 Gew.-% Isobuten enthält.

4. Verfahren nach einem der Ansprüche 2 und 3, dadurch gekennzeichnet, daß der n-Butan-Schnitt, der im Verlauf der in Stufe (i) durchgeführten Fraktionierung gesammelt wird, in eine Zone zur Crackung mit Dampf geführt wird.

5. Verfahren nach einem der Ansprüche 2 bis 4, dadurch gekennzeichnet, daß der C$_4$-olefinische Schnitt, der erfindungsgemäß behandelt wurde, aus einer Zone der Crackung mit Dampf hervorgeht und daß der n-Butan-Schnitt, der im Verlaufe der in Stufe (i) durchgeführten Fraktionierung erhalten wird, in diese Zone der Crackung mit Dampf zurückgeführt wird.

6. Verfahren nach Anspruch 1 zur Erzielung von Buten-1 hoher Reinheit, ausgehend von einem olefinischen C$_4$-Schnitt, dadurch gekennzeichnet, daß (siehe Figur 2)

a) ein olefinischer C$_4$-Schnitt, der Isobutan, n-Butan, Buten-1 und Butene-2, Isobuten und weniger als 2 Gew.-% Butadien enthält, einer Polymerisation-Disproportionierung in einer Zone (4) unterworfen wird, die in flüssiger Phase bewirkt wird, und im Verlaufe welcher mindestens 95 Gew.-% des Isobutens reagieren und sich in einen Treibstoff umwandeln, der im wesentlichen aus Dimeren und Trimeren des Isobutens besteht und ein Teil dieses Treibstoffes sich einerseits in Butene-2 und Buten-1 und andererseits in einen Schnitt vom Typ Düsentreibstoffbasis auf der Basis der Trimeren und Tetrameren des Isobutens umwandelt;

b) der aus der Polymerisations-Disproportionierungszone (4) abgezogene Abstrom einer Fraktionierung in der Zone (6) unterworfen wird, im Verlaufe welcher man einerseits ein Gemisch ($\alpha$) von Treibstoff und Düsentreibstoffbasis gewinnt und andererseits eine Fraktion ($\beta$), die im wesentlichen aus Isobutan, n-Butan, Buten-1 und Butenen-2, weniger als 5 Gew.-% Isobuten und weniger als 2000 ppm Butadien besteht;

c) die in Stufe (b) erhaltene Fraktion ($\beta$) in eine Zone (8) der selektiven Polymerisation, die Fertigstellungspolymerisation genannt wird, überführt wird, worin wenigstens 90 Gew.-% des Isobutens, das sie enthält, in Treibstoff überführt werden, während die Gesamtumwandlung der Butene-2 und des Buten-1 unter oder gleich 10 Gew.-% bleibt;

d) der Abstrom der Fertigstellungspolymerisationszone einer Fraktionierung [Zone (10)] unterworfen wird, im Verlaufe welcher man einerseits eine Fraktion ($\gamma$) gewinnt, die zum größeren Teil aus Treibstoff besteht, der die Dimeren und die Trimeren des Isobutans und der Butene-1 und -2 enthält, und andererseits eine Fraktion ($\delta$), die im wesentlichen aus Isobutan, n-Butan, Buten-1

21

und Butenen-2, weniger als 0,3 Gew.-% Isobuten und weniger als 100 ppm Butadien besteht;

e) die Fraktion ($\delta$), die aus der Stufe (d) stammt, in eine Zone (20) zur selektiven Hydrierung geführt wird, derart, daß sich ihr Gehalt an Butadien auf weniger als 10 Teile pro Million, bezogen auf in der Fraktion ($\delta$) vorhandenem Buten-1 vermindert;

f) der Abstrom der Stufe (e) in eine Zone (22) zur Deisobutanisierung geführt wird, um den größeren Teil des Isobutans, das es enthält, zu beseitigen;

g) die so deisobutanisierte Fraktion in der Zone (25) einer Fraktionierung unterworfen wird, im Verlaufe welcher man einerseits einen Schnitt gewinnt, der einen größeren Teil der Butene-2 und n-Butan enthält, und andererseits eine Fraktion, die aus wenigstens 99 Gew.-% Buten-1 besteht; und

h) die Mischung ($\alpha$), welche aus dem Treibstoff und der Düsentreibstoffbasis besteht, und aus der Stufe (b) stammt, mit der Treibstoff-Fraktion ($\gamma$) aus der Stufe (d) gemischt wird, und die neue Mischung dann in der Zone (15) einer Fraktionierung unterzogen wird, im Verlaufe welcher man einerseits einen Treibstoffschnitt von hoher Octanzahl und andererseits einen Schnitt für Düsentreibstoff sammelt.

7. Verfahren nach Anspruch 6, dadurch gekennzeichnet, daß die Fraktion ($\beta$) außerdem weniger als 2 Gew.-% Isobuten und weniger als 500 ppm Butadien enthält, und daß die Fraktion ($\delta$) weniger als 0,15 Gew.-% Isobuten enthält.

8. Verfahren nach Anspruch 6, dadurch gekennzeichnet, daß außerdem dieser als Düsentreibstoff verwendbare Schnitt, der am Ende der Fraktionierung in der Stufe (h) erhalten ist, in eine Hydrierungszone überführt wird, bevor er als Brennstoff verwendet wird.

9. Verfahren nach einem der Ansprüche 6 bis 8, dadurch gekennzeichnet, daß die selektive Hydrierung in der Stufe (e) in Gegenwart eines auf einen Träger aufgebrachten Katalysators durchgeführt wird, der 0,05 bis 0,5 Gew.-% Palladium und 0,05 bis 1 Gew.-% Silber enthält, wobei das Gewichtsverhältnis Silber/Palladium 0,7 : 1 bis 3 : 1 ist, und der Katalysatorträger ein Aluminiumoxid mit einer Oberfläche von 1 bis 100 $m^2/g$ ist.

10. Verfahren nach einem der Ansprüche 6 bis 9, dadurch gekennzeichnet, daß die Polymerisation-Disproportionierung der Stufe (a) und die Fertigstellungspolymerisation der Stufe (c) in Gegenwart eines Siliziumdioxid-Aluminiumoxid-Katalysators durchgeführt werden.

11. Verfahren nach Anspruch 10, dadurch gekennzeichnet, daß der Katalysator zur Polymerisierung-Disproportionierung außerdem 0,1 bis 5 Gew.-% Chromoxid enthält und der Katalysator zur Fertigstellungspolymerisation außerdem 0,1 bis 5 Gew.-% Zinkoxid enthält.

12. Verfahren nach Anspruch 1 zur gemeinsamen Herstellung von Buten-1 hoher Reinheit, Supertreibstoff und Düsentreibstoff, ausgehend von einem olefinischen $C_4$-Schnitt, das darin besteht, daß (siehe Figur 3):

a) eine frische Beschickung, die im wesentlichen aus einem olefinischen $C_4$-Schnitt besteht, der Isobutan, n-Butan, Buten-1, Butene-2, Isobuten und weniger als 2 Gew.-% Butadien enthält, einer Polymerisations-Disproportionierung in einer Zone (4) in flüssiger Phase unterworfen wird, im Verlaufe welcher einerseits ein Teil des Isobutens dieses Schnittes im wesentlichen mindestens teilweise in einen Treibstoff (Dimere und Trimere von Isobuten) überführt und andererseits ein Teil des so gebildeten Treibstoffes zumindest teilweise einer partiellen Disproportionierungsreaktion derart unterworfen wird, daß man einen Schnitt einer Düsentreibstoffbasis (Gemisch von Trimeren und Tetrameren des Isobutens) und Butene-2 und vor allem Buten-1 sammelt;

b) der von der Polymerisations-Disproportionierungszone (4) abgezogene Abstrom einer Fraktionierung in Zone (6) unterworfen wird, im Verlaufe welcher man einerseits ein Gemisch ($\alpha$) an Treibstoff und Düsentreibstoffbasis gewinnt und andererseits eine Fraktion ($\beta$), die im wesentlichen aus Isobutan, n-Butan, Buten-1, Butenen-2, weniger als 5 Gew.-% Isobuten und weniger als 2000 Gew.-ppm Butadien besteht;

c) die in Stufe (b) erhaltene Fraktion ($\beta$) in eine Zone (8) der selektiven Polymerisation gebracht wird, die Fertigstellungs-Polymerisation genannt wird, worin mindestens 90 Gew.-% des Isobutens, das sie enthält, in Treibstoff überführt werden, wobei die selektive Polymerisationsreaktion in flüssiger Phase bewirkt wird, und die Gesamtumwandlung der Butene-2 und des Buten-1 unter oder gleich 10 Gew.-% bleibt;

d) der Abstrom der Fertigstellungspolymerisationszone einer Fraktionierung [Zone (10)] unterworfen wird, im Verlaufe welcher man einerseits eine Fraktion ($\gamma$) gewinnt, die größtenteils aus Treibstoff besteht, der insbesondere die Dimeren und die Trimeren des Isobutens und der Butene-1 und -2 enthält und andererseits eine Fraktion ($\delta$), die im wesentlichen aus Isobutan, n-Butan, Buten-1 und Butenen-2, weniger als 0,3 Gew.-% Isobuten und weniger als 100 Gew.-ppm Butadien besteht;

e) die Fraktion ($\delta$), die aus Stufe (d) kommt, in eine Zone (20) geleitet wird, zur selektiven Hydrierung derart, daß sich der Gehalt an Butadien auf weniger als 10 Gew.-Teile pro Million, bezogen auf in der Fraktion ($\delta$) vorhandenes Buten-1, vermindert;

f) der Abstrom der Stufe (e) in eine Zone (22) zur Deisobutanisierung geführt wird, um zumindest den größten Teil des Isobutans zu entfernen, das er enthält;

**0 068 981**

g)  die so deisobutanisierte Fraktion einer Fraktionierung in der Zone (25) unterworfen wird, im Verlaufe welcher man einerseits einen Schnitt (ε) gewinnt, der den größeren Teil der Butene-2 und des n-Butans enthält, und andererseits eine Fraktion, die aus mindestens 99 Gew.-% Buten-1 besteht, wobei das Verfahren dadurch gekennzeichnet ist, daß man anschließend

h)  diesen Schnitt (ε), der den größeren Teil der Butene-2 und des n-Butans enthält und aus Stufe (g) stammt, durch eine Leitung (27) zu einer Isomerisierungszone (28) führt, worin zumindest ein Teil der Butene-2 in Gasphase isomerisiert wird zu Isobuten und Buten-1, ohne den größeren Teil des n-Butans zu berühren;

i)  das aus der Isomerisierungszone der Stufe (h) stammende Produkt dann in eine Zone (30) zur Fraktionierung geführt wird, bei welcher man am Boden eine Fraktion gewinnt, die vor allem Buten-1, die Butene-2, Isobuten, Isobutan, n-Butan und den Treibstoff enthält, wobei diese Fraktion zu einer Zone (33) zur Fraktionierung geführt wird, an deren Boden man den Treibstoff gewinnt und wo man am Kopf eine Mischung abzieht, die insbesondere Isobuten, Buten-1, die Butene-2 und Isobutan enthält;

j)  diese in der vorhergehenden Stufe am Kopf von der Fraktionierungszone (33) erhaltene Mischung in eine Zone (36) der extraktiven Lösungsmitteldestillation führt derart, daß man ein Gemisch sammelt, das insbesondere Isobuten, Buten-1 und die Butene-2 enthält, die man zum Eingang der Zone (4) der Polymerisation-Disproportionierung zurückführt; und

k)  das Gemisch (α) von Treibstoff und Düsentreibstoffbasis, das von der Stufe (b) stammt, einerseits mit dem Treibstoff vereinigt, den man am Boden der Zone (33) der Fraktionierung in der Stufe (i) erhält, und andererseits mit der Fraktion (γ), die man in Stufe (d) erhalten hat, und das Gemisch dann in eine Zone (15) der Fraktionierung führt, am Kopf welcher man einen Supertreibstoff gewinnt, und am Boden welcher man einen Brennstoff für Düsentriebwerke gewinnt.

13. Verfahren nach Anspruch 12, dadurch gekennzeichnet, daß man den Düsentreibstoff, der in Stufe (k) erhalten wird, dann einer Hydrierung unterwirft, im Verlaufe welcher dieser Teile der Butene-2 in Gegenwart von Wasserdampf isomerisiert wird, wobei das molare Verhältnis von Wasserdampf zu Kohlenwasserstoffen zwischen 0,2 und 2 liegt.

14. Verfahren nach Anspruch 13, dadurch gekennzeichnet, daß man die Butene-2 in Gegenwart eines Katalysators auf der Basis eines fluorierten Aluminiumoxides isomerisiert.

15. Verfahren nach einem der Ansprüche 13 und 14, dadurch gekennzeichnet, daß man im Verlaufe der Isomerisierung zumindest 40% und vorzugsweise zumindest 45% der Butene-2 in Isobuten und Buten-1 überführt, während zumindest 80%, vorzugsweise zumindest 90%, des n-Butans nicht berührt werden.

**Claims**

1. A process for producing highly pure 1-butene and premium gasoline from $C_4$ olefinic cut, characterized in that:

a)  a $C_4$ olefinic cut which, generally, at this stage, contains isobutane, n-butane, 1-butene, 2-butenes, isobutene and less than 2% by weight of butadiene, is subjected to a polymerization disproportionation in the liquid phase, in the presence of at least an acid catalyst (containing at least one compound selected from the group consisting of the silica-aluminas, the silica-magnesiae, the boron-aluminas, phosphoric acid deposited on Kieselguhr or silica or quartz, catalysts of the solid phosphoric type and transition aluminas treated with at least one acid fluorine compound), between 10 to 120°C, under a pressure from 0.3 to 10 MPa, with a liquid hydrocarbon flow rate (space velocity) of about 0.05 to 2 volumes per volume of catalyst and per hour, during which, on the one hand, 1- and 2-butenes remain substantially unconverted, and the isobutene of said cut is converted at least partly to gasoline and, on the other hand, the so-produced gasoline is subjected at least partly to a reaction of partial disproportionation so as to recover a cut of the jet fuel base type and 2-butenes, and mainly 1-butene, produced by said disproportionation,

b)  the effluent withdrawn from the polymerization-disproportionation zone is subjected to a fractionation giving, on the one hand, a mixture (α) of gasoline and jet fuel base and, on the other hand, a fraction (β) consisting essentially of isobutane, n-butane, 1-butene, 2-butenes, less than 5% by weight of isobutene and less than 2000 ppm of butadiene,

c)  the cut (β) obtained in step (b) is fed to a selective polymerization zone called finishing polymerization zone, wherein more than 90% of the residual isobutene is converted to gasoline, whereas the total conversions of the normal butenes remain lower or equal to 10% by weight, this finishing polymerization reacting being carried out in liquid phase, in the presence of at least an acid catalyst which is defined as the above polymerization-disproportionation catalyst, at a temperature of about 30 to 200°C, under a pressure from about 0.5 to 20 MPa, with a liquid hydrocarbon flow rate (space velocity) of about 0.2 to 4 volumes per volume of catalyst and per hour,

d)  the effluent withdrawn from said finishing polymerization zone of step (c) is subjected to a

23

fractionation giving, on the one hand, a cut ($\gamma$) consisting in major part of gasoline containing in particular a mixture of dimers and trimers of isobutene and of 1- and 2-butenes and, on the other hand, a fraction ($\delta$) consisting essentially of isobutane, n-butane, 1-butene, 2-butenes, less than 0.3% by weight of isobutene and less than 100 parts per million of butadiene,

e) the cut ($\delta$) issued from step (d) is fed to a selective hydrogenation zone carried out at a temperature between 10 and 100°C, under a pressure between 0.1 and 3 MPa, with a liquid hydrocarbon flow rate (space velocity) of from about 2 to 20 volumes per volume of catalyst and per hour, the hydrogen/butadiene molar ration, at the reactor input, being from 0.5 to 5, so as to reduce the butadiene content of said cut ($\delta$) to a maximum of 10 parts per million by weight with respect to the 1-butene of said cut ($\delta$),

f) the effluent from the selective hydrogenation step (e) is supplied to a deisobutanization zone in order to remove the major part of the isobutane contained therein,

g) the so-deisobutanized fraction is subjected to a fractionation giving on the one hand, a cut containing a major portion of 2-butenes and n-butane and, on the other hand, a fraction containing at least 99% by weight of 1-butene, and the mixture ($\alpha$) formed by the gazoline and the jet fuel base obtained in step (b) is admixed with the gazoline cut ($\gamma$) obtained in step (d).

2. A process according to claim 1 for producing highly pure 1-butene and premium gazoline from a $C_4$ olefinic cut characterized in that (see figure 1):

a) a $C_4$ olefinic cut containing isobutane, n-butane, 1-butene, 2-butenes, isobutene and less than 2% by weight of butadiene is subjected in a zone (4) to a polymerization-disproportionation, effected in liquid phase, during which at least 95% of the isobutene of said cut is essentially converted to gasoline essentially consisting of dimers and trimers of isobutene, a portion of said gasoline being converted on the one hand to 2-butenes and 1-butene and on the other hand to a jet fuel base type cut containing trimers and tetramers of isobutene,

b) the effluent withdrawn from the polymerization-disproportionation zone (4) is subjected to a fractionation in zone (6) giving, on the one hand, a mixture ($\alpha$) of gasoline and jet fuel base and, on the other hand, a fraction ($\beta$) essentially consisting of isobutane, n-butane, 1-butene, 2-butenes, less than 5% by weight of isobutene and less than 2000 ppm of butadiene,

c) the fraction ($\beta$) obtained in step (b) is fed to a selective, called finishing polymerization zone (8) wherein 90% at least, by weight, of the isobutene contained therein are converted to gasoline, whereas the total conversions of 2-butenes and 1-butene remain lower or equal to 10% by weight,

d) the effluent from the finishing polymerization zone in subjected to a fractionation (zone 10) during which is recovered, on the one hand, a cut ($\gamma$) consisting in major part of gasoline containing dimers and trimers of isobutene and of 1- and 2-butenes and, on the other hand, a fraction ($\delta$) essentially consisting of isobutane, n-butane, 1-butene, 2-butenes, less than 0.3% by weight of isobutene and less than 100 ppm of butadiene,

e) the cut ($\delta$) obtained in step (d) is fed to a selective hydrogenation zone (20) so as to reduce it butadiene content to less than 10 parts per million with respect to the 1-butene present in said cut ($\delta$),

f) the effluent from step (e) is fed to a deisobutanization zone (22) in order to remove the major part of the isobutane contained therein,

g) the so deisobutanized fraction is subjected to a fractionation (zone 25) from which is recovered, on the one hand, a cut containing mainly 2-butenes and n-butane and, on the other hand, a fraction containing at least 99% by weight of 1-butene,

h) the mixture ($\alpha$) formed of gasoline and jet fuel base obtained in step (b) is admixed with the gasoline cut ($\gamma$) obtained in step (d) and with the $C_4$ cut, of high 2-butenes and n-butane content, obtained in step (g), the new resulting mixture being subjected to hydrogenation in zone (15),

i) the hydrogenation effluent of step (h) is subjected to at least one fractionation from which is recovered n-butane, a gasoline cut and a fuel cut.

3. A process according to claim 2, wherein, said cut ($\beta$) further contains, by weight, less than 2% of isobutene and less than 500 ppm of butadiene, and wherein said cut ($\delta$) contains less than 0.15% by weight of isobutene.

4. A process according to one of claims 2 and 3, wherein the n-butane cut recovered during the fractionation of step (i) is fed to a steam-cracking zone.

5. A process according to one of claims 2 to 4, wherein the olefinic $C_4$ cut, treated according to the invention, is issued from a steam-cracking zone and wherein the n-butane cut recovered during the fractionation of step (i) is recycled to said steam-cracking zone.

6. A process according to claim 1 for producing 1-butene of high purity from a $C_4$ olefinic cut, characterized in that (see figure 2):

a) a $C_4$ olefinic cut containing isobutane, n-butane, 1-butene, 2-butenes, isobutene and less than 2% by weight of butadiene, is subjected in a zone (4) to a polymerization-disproportionation, effected in liquid phase, during which, at least 95% by weight of the isobutene reacts and is converted to a gasoline essentially formed of isobutene dimers and trimers, a portion of said gasoline being converted, on the one hand, to 2-butenes and 1-butene and, on the other hand, to a jet fuel base type cut, comprising isobutene trimers and tetramers,

b) the effluent withdrawn from the polymerization-disproportionation zone (4) is subjected to a fractionation in zone (6) giving, on the one hand, a mixture ($\alpha$) of gasoline and jet fuel base and, on the other hand, a fraction ($\beta$) consisting essentially of isobutane, n-butane, 1-butene, 2-butenes, less than 5% by weight of isobutene and less than 2000 ppm of butadiene,

c) the fraction ($\beta$) obtained in step (b) is fed to a selective, called finishing polymerization zone (8) wherein at least 90% by weight of the isobutene contained therein is converted to gasoline, whereas the total conversions of 2-butenes and 1-butene remain smaller or equal to 10% by weight,

d) the effluent from the finishing polymerization zone is subjected to a fractionation (zone 10) giving, on the one hand, a fraction ($\gamma$) consisting in major part of gasoline, containing dimers and trimers of isobutene and of 1- and 2-butenes and, on the other hand, a fraction ($\delta$) consisting essentially of isobutane, n-butane, 1-butene, 2-butenes, less than 0.3% by weight of isobutene and less than 100 ppm of butadiene,

e) the fraction ($\delta$) issued from step (d) is fed to a selective hydrogenation zone (20), so as to reduce its butadiene content to less than 10 parts per million with respect to the 1-butene present in said fraction ($\delta$),

f) the effluent from step (e) is fed to a deisobutanization zone (22) in order to remove the major part of the isobutane contained therein,

g) the so-deisobutanized fraction is subjected, in zone (25), to a fractionation giving, on the one hand, a cut containing in major part 2-butenes and n-butane and, on the other hand, a fraction consisting of at least 99% by weight of 1-butene, and

h) the mixture ($\alpha$) consisting of gasoline and jet fuel base obtained in step (b) is admixed with the gasoline fraction ($\gamma$) obtained in step (d), the resulting new mixture being subjected, in zone (15), to a fractionation giving, on the one hand, a gasoline cut of high octane number and, on the other hand, a jet fuel cut.

7. A process according to claim 6, wherein the fraction ($\beta$) further contains, by weight, less than 2% of isobutene and less than 500 ppm of butadiene, and wherein the fraction ($\delta$) contains less than 0.15% by weight of isobutene.

8. A process according to claim 6, wherein, in addition, said jet fuel cut obtained at the end of the fractionation of step (h) is fed to a hydrogenation zone before being used as fuel.

9. A process according to any one of claims 6 to 8, wherein the selective hydrogenation of step (e) is effected in the presence of a supported catalyst containing 0.05 to 0.5% by weight of palladium and from 0.05 to 1% by weight of silver, the ratio by weight silver/palladium being from 0.7 : 1 to 3 : 1, the catalyst carrier being an alumina of a specific surface from 1 to 100 $m^2$/g.

10. A process according to any one of claims 6 to 9, wherein the polymerization-disproportionation of step (a) and the finishing polymerization of step (c) are effected in the presence of a silica-alumina catalyst.

11. A process according to claim 10, wherein the polymerization-disproportionation catalyst further contains 0.1 to 5% by weight of chromium oxide and the finishing polymerization catalyst further contains 0.1 to 5% by weight of zinc oxide.

12. A process according to claim 1, for the joint production of highly pure 1-butene, premium gasoline and jet fuel, from a $C_4$ olefinic cut, wherein (see figure 3):

a) a fresh charge consisting essentially of $C_4$ olefinic cut containing isobutane, n-butane, 1-butene, 2-butenes, isobutene and less than 2% by weight of butadiene, is subjected, in a zone (4), to a polymerization-disproportionation in liquid phase, during which on the one hand, the isobutene of said cut is essentially converted, at least partly to gasoline (dimers and trimers of isobutene), and on the other hand, the obtained gasoline is subjected, at least partly, to a disproportionation so as to recover, a jet fuel base type cut (mixture of trimers and tetramers of isobutene), and 2-butenes and mainly 1-butene,

b) the effluent withdrawn from the polymerization-disproportionation zone (4) is subjected in zone (6) to a fractionation giving, on the one hand, a mixture ($\alpha$) of gasoline and jet fuel base and, on the other hand, a fraction ($\beta$) essentially consisting of isobutane, n-butane, 1-butene, 2-butenes, less than 5% by weight of isobutene and less than 2000 ppm by weight of butadiene,

c) the fraction ($\beta$) obtained in step (b) is fed to a selective polymerization zone (8) called finishing-polymerization zone, wherein at least 90% by weight of the isobutene contained therein are converted to gasoline, the selective polymerization reaction being effected in liquid phase, the total conversion of the 2-butenes and 1-butene being lower than or equal to 10% by weight,

d) the effluent from the finishing polymerization zone is subjected to a fractionation (zone 10) giving, on the one hand, a fraction ($\gamma$) consisting in major part of gasoline, containing in particular dimers and trimers of isobutene and of 1- and 2-butenes, and on the other hand, a fraction ($\delta$) consisting essentially of isobutane, n-butane, 1-butene, 2-butenes, less than 0.3% by weight of isobutene and less than 100 ppm by weight of butadiene,

e) the fraction ($\delta$) issued from step (b) is fed to a selective hydrogenation zone (20) so as to reduce its butadiene content to less than 10 parts per million by weight with respect to the 1-butene present in said fraction ($\delta$),

f) the effluent from step (e) is fed to a deisobutanization zone (22) inorder to remove at least the major part of the isobutane contained therein,

g) the so-deisobutanized fraction is subjected to a fractionation, in zone (25) giving, on the one hand, a cut ($\varepsilon$) containing in major part 2-butenes and n-butane and, on the other hand, a fraction consisting of at least 99% by weight of 1-butene, the process being characterized in that, thereafter,

h) said cut ($\varepsilon$) containing in major part 2-butenes and n-butane, issued from step (g), is fed through a line (27) to an isomerization zone (28) wherein at least a portion of said 2-butenes is isomerized in gaseous phase to isobutene and to 1-butene, while maintaining unchanged the major part of the n-butane,

i) the product issued from the isomerization zone of step (h) is first fed to a fractionation zone (30) from the bottom of which is recovered a fraction containing mainly 1-butene, 2-butenes, isobutene, isobutane, n-butane and gasoline, said fraction being fed to a fractionation zone (33) from the bottom of which is recovered gasoline and at the top of which is withdrawn a mixture containing particularly isobutene, 1-butene, 2-butenes and isobutane,

j) said mixture, obtained in the preceding step at the top of the fractionation zone (33), is fed to a solvent extraction distillation zone (36) so as to recover a mixture containing mainly isobutene, 1-butene and 2-butenes which is recycled to the input of the polymerization-disproportionation zone (4), and

k) the mixture ($\alpha$) of gasoline and jet fuel base issued from step (b) is combined, on the one hand, with the gasoline obtained at the bottom of the fractionation zone (33) of step (i) and, on the other hand, with the fraction ($\gamma$) obtained in step (d), the resultant mixture being fed to a fractionation zone (15) at the top of which is recovered a premium gasoline and from the bottom of which is recovered a jet fuel.

13. A process according to claim 12, wherein the jet fuel obtained in step (k) is then subjected to a hydrogenation, wherein, said portion of 2-butenes is isomerized in the presence of steam, the molar ration steam/hydrocarbons being from 0.2 to 2.

14. A process according to claim 13, wherein, in addition, the 2-butenes are isomerized in the presence of a catalyst containing fluorinated alumina.

15. A process according to one of claims 13 and 14, wherein, during the isomerization, at least 40% and preferably at least 45% of the 2-.butenes are converted to isobutene and 1-butene, whereas at least 80%, preferably at least 90% of the n-butane remain unchanged.

FIG.1

FIG.2

FIG.3